# EUROPEAN PATENT APPLICATION

(11) **EP 2 366 432 A1**
(43) Date of publication of application: **21.09.2011**
(21) Application number: 10002735.8
(22) Date of filing: 16.03.2010
(51) Int. Cl.: A61P 11/00, A61K 31/404, A61K 31/519, A61K 31/4015

(54) **Inhibitors of glycogen synthase kinase 3 for use in therapeutic methods and screening method relating thereto**

(71) Applicant: Helmholtz Zentrum München Deutsches Forschungszentrum für Gesundheit und Umwelt (GmbH), 85764 Neuherberg (DE)
(72) Inventor: Königshoff, Melanie, Dr. Dr., 80789 München (DE); Eickelberg, Oliver, Prof. Dr., 35392 Giessen (DE)
(74) Representative: Bösl, Raphael Konrad

(57) **Abstract**

The present invention relates to an inhibitor of glycogen synthase kinase 3 (GSK-3) for use in a method of treating or preventing emphysema, a method of activating an emphysema cell, an activated emphysema cell obtained by this method for use in a method of treating or preventing emphysema, and a method of screening for a substance for treating or preventing emphysema.

SEQUENCE LISTING

<110> Helmholtz Zentrum München Deutsches Forschungszentrum für
Gesundheit und Umwelt (GmbH)

<120> Inhibitors of Glycogen Synthase Kinase 3 for Use in Therapeutic
Methods and Screening Method Relating Thereto

<130> H67111EP

<160> 66

<170> PatentIn version 3.5

<210> 1
<211> 24
<212> DNA
<213> Artificial Sequence

<220>
<223> Primer

<400> 1
aagtgggtgg tatagaggct cttg 24

<210> 2
<211> 21
<212> DNA
<213> Artificial Sequence

<220>
<223> Primer

<400> 2
gatggcaggc tcagtgatgt c 21

<210> 3
<211> 17
<212> DNA
<213> Artificial Sequence

<220>
<223> Primer

<400> 3
agcgccgtgg agttcgt 17

<210> 4
<211> 27
<212> DNA
<213> Artificial Sequence

<220>
<223> Primer

<400> 4
cgaaagagag ttgtctagtg aggaaac 27

<210> 5
<211> 15
<212> DNA
<213> Artificial Sequence

<220>
<223> Primer

<400>  5
cacgccgcgc atgtc                                                        15


<210>  6
<211>  23
<212>  DNA
<213>  Artificial Sequence

<220>
<223>  Primer

<400>  6
acgatgagcg tcatgaggta ttt                                               23


<210>  7
<211>  20
<212>  DNA
<213>  Artificial Sequence

<220>
<223>  Primer

<400>  7
ggtgttcctt ggcctgaaga                                                   20


<210>  8
<211>  23
<212>  DNA
<213>  Artificial Sequence

<220>
<223>  Primer

<400>  8
cacaagtcga ggatatggct cat                                               23


<210>  9
<211>  22
<212>  DNA
<213>  Artificial Sequence

<220>
<223>  Primer

<400>  9
gacaactttc acaccgctca tc                                                22


<210>  10
<211>  19
<212>  DNA
<213>  Artificial Sequence

<220>
<223>  Primer

<400>  10
ccttcaggac gggttcaca                                                    19


<210>  11
<211>  20
<212>  DNA
<213>  Artificial Sequence

<220>
<223>  Primer

<400>  11
ctcatgctcg gattcaagca                                                    20


<210>  12
<211>  22
<212>  DNA
<213>  Artificial Sequence

<220>
<223>  Primer

<400>  12
ggtctgtcca cggtctccag ta                                                 22


<210>  13
<211>  24
<212>  DNA
<213>  Artificial Sequence

<220>
<223>  Primer

<400>  13
catcaggtac aggtccaaga atga                                               24


<210>  14
<211>  18
<212>  DNA
<213>  Artificial Sequence

<220>
<223>  Primer

<400>  14
gtcgctgcct tggctttg                                                      18


<210>  15
<211>  21
<212>  DNA
<213>  Artificial Sequence

<220>
<223>  Primer

<400>  15
gattcagatc tccggcgaat t                                                  21


<210>  16
<211>  24
<212>  DNA
<213>  Artificial Sequence

<220>
<223>  Primer

<400>  16
ggctgcaaga tattggagtc ttct                                               24


<210>  17

<211>  26
<212>  DNA
<213>  Artificial Sequence

<220>
<223>  Primer

<400>  17
accatctcca gcacacttgt ctaata                                          26


<210>  18
<211>  20
<212>  DNA
<213>  Artificial Sequence

<220>
<223>  Primer

<400>  18
gagtcagcgg atgcatgtga                                                 20


<210>  19
<211>  21
<212>  DNA
<213>  Artificial Sequence

<220>
<223>  Primer

<400>  19
gcgcgggata actatggaaa g                                               21


<210>  20
<211>  24
<212>  DNA
<213>  Artificial Sequence

<220>
<223>  Primer

<400>  20
ggatttagga aacattcgct gtgt                                            24


<210>  21
<211>  25
<212>  DNA
<213>  Artificial Sequence

<220>
<223>  Primer

<400>  21
cctgatgaat cttcacaaca acaga                                           25


<210>  22
<211>  19
<212>  DNA
<213>  Artificial Sequence

<220>
<223>  Primer

<400>  22
ccgtggcact tgcactctt                                                  19

<210>  23
<211>  23
<212>  DNA
<213>  Artificial Sequence

<220>
<223>  Primer

<400>  23
gccccactcg gatacttctt act                                              23


<210>  24
<211>  21
<212>  DNA
<213>  Artificial Sequence

<220>
<223>  Primer

<400>  24
gaggaatact gtggcccaac a                                                21


<210>  25
<211>  25
<212>  DNA
<213>  Artificial Sequence

<220>
<223>  Primer

<400>  25
gcaagtggat tttctacgtg tttct                                            25


<210>  26
<211>  20
<212>  DNA
<213>  Artificial Sequence

<220>
<223>  Primer

<400>  26
tgacagtgct ccgagcttca                                                  20


<210>  27
<211>  19
<212>  DNA
<213>  Artificial Sequence

<220>
<223>  Primer

<400>  27
gcgccaggtg gtaactgaa                                                   19


<210>  28
<211>  19
<212>  DNA
<213>  Artificial Sequence

<220>
<223>  Primer

<400>  28
tgcctgatgt gccatgaca                                                    19


<210>  29
<211>  20
<212>  DNA
<213>  Artificial Sequence

<220>
<223>  Primer

<400>  29
aaggacccca cgaagtgttg                                                   20


<210>  30
<211>  22
<212>  DNA
<213>  Artificial Sequence

<220>
<223>  Primer

<400>  30
ggctttgtat tttgcttttc ca                                                22


<210>  31
<211>  24
<212>  DNA
<213>  Artificial Sequence

<220>
<223>  Primer

<400>  31
tcaagagagc aagctcatca ttct                                              24


<210>  32
<211>  22
<212>  DNA
<213>  Artificial Sequence

<220>
<223>  Primer

<400>  32
caccttcagc actctgcttg tg                                                22


<210>  33
<211>  20
<212>  DNA
<213>  Artificial Sequence

<220>
<223>  Primer

<400>  33
ccttatccat tggcttgtcg                                                   20


<210>  34
<211>  20
<212>  DNA
<213>  Artificial Sequence

<220>
<223>  Primer

<400>  34
ctgaaccgat tcatgaccac                                                   20


<210>  35
<211>  19
<212>  DNA
<213>  Artificial Sequence

<220>
<223>  Primer

<400>  35
ccatcctcgg aatccttgg                                                    19


<210>  36
<211>  20
<212>  DNA
<213>  Artificial Sequence

<220>
<223>  Primer

<400>  36
tttgaccacc gttctcctcc                                                   20


<210>  37
<211>  23
<212>  DNA
<213>  Artificial Sequence

<220>
<223>  Primer

<400>  37
ccaagaagac atccctgaag tca                                               23


<210>  38
<211>  18
<212>  DNA
<213>  Artificial Sequence

<220>
<223>  Primer

<400>  38
tgcacgtcat cgcacaca                                                     18


<210>  39
<211>  22
<212>  DNA
<213>  Artificial Sequence

<220>
<223>  Primer

<400>  39
aaacagcaca ggttctgcaa aa                                                22


<210>  40

<211>  17
<212>  DNA
<213>  Artificial Sequence

<220>
<223>  Primer

<400>  40
tgggccctct cgttctt                                                    17


<210>  41
<211>  21
<212>  DNA
<213>  Artificial Sequence

<220>
<223>  Primer

<400>  41
tccatctggt gggtgattct g                                               21


<210>  42
<211>  19
<212>  DNA
<213>  Artificial Sequence

<220>
<223>  Primer

<400>  42
ctcgtggccc cacttcatt                                                  19


<210>  43
<211>  23
<212>  DNA
<213>  Artificial Sequence

<220>
<223>  Primer

<400>  43
tttgagctgg atccctagga tga                                             23


<210>  44
<211>  19
<212>  DNA
<213>  Artificial Sequence

<220>
<223>  Primer

<400>  44
ttcttcgctt tccgatgca                                                  19


<210>  45
<211>  17
<212>  DNA
<213>  Artificial Sequence

<220>
<223>  Primer

<400>  45
ggcggcgttg gacagat                                                    17

<210>  46
<211>  21
<212>  DNA
<213>  Artificial Sequence

<220>
<223>  Primer

<400>  46
cacccgtgat gggataaaca g                                                21


<210>  47
<211>  24
<212>  DNA
<213>  Artificial Sequence

<220>
<223>  Primer

<400>  47
caacgtggac gtgttttatt cttc                                             24


<210>  48
<211>  24
<212>  DNA
<213>  Artificial Sequence

<220>
<223>  Primer

<400>  48
cagcgactgg tggtgctgta gtca                                             24


<210>  49
<211>  22
<212>  DNA
<213>  Artificial Sequence

<220>
<223>  Primer

<400>  49
ccattcctct cactggtgtc aa                                               22


<210>  50
<211>  23
<212>  DNA
<213>  Artificial Sequence

<220>
<223>  Primer

<400>  50
gccaaactct accacatgtt cca                                              23


<210>  51
<211>  22
<212>  DNA
<213>  Artificial Sequence

<220>
<223>  Primer

<400>  51
ggtacaaggc tttcacgatc gc                                                22

<210>  52
<211>  18
<212>  DNA
<213>  Artificial Sequence

<220>
<223>  Primer

<400>  52
atgtccggta acggcggc                                                     18

<210>  53
<211>  19
<212>  DNA
<213>  Artificial Sequence

<220>
<223>  Primer

<400>  53
tctgctcatg ggcctccac                                                    19

<210>  54
<211>  19
<212>  DNA
<213>  Artificial Sequence

<220>
<223>  Primer

<400>  54
cgatggtgtc tgctcgctc                                                    19

<210>  55
<211>  18
<212>  DNA
<213>  Artificial Sequence

<220>
<223>  Primer

<400>  55
gtgggaactg ccccgttt                                                     18

<210>  56
<211>  22
<212>  DNA
<213>  Artificial Sequence

<220>
<223>  Primer

<400>  56
gttcctgatg aaccttcaca ac                                                22

<210>  57
<211>  21
<212>  DNA
<213>  Artificial Sequence

<220>
<223>  Primer

<400>  57
agcttccgaa gccgaagtat c                                                21

<210>  58
<211>  21
<212>  DNA
<213>  Artificial Sequence

<220>
<223>  Primer

<400>  58
agaacggagt cgtgtgcttt g                                                21

<210>  59
<211>  22
<212>  DNA
<213>  Artificial Sequence

<220>
<223>  Primer

<400>  59
agccctgatg aaccttcaca ac                                               22

<210>  60
<211>  24
<212>  DNA
<213>  Artificial Sequence

<220>
<223>  Primer

<400>  60
tgacacttgc attcttgttt caag                                             24

<210>  61
<211>  18
<212>  DNA
<213>  Artificial Sequence

<220>
<223>  Primer

<400>  61
gcaccaccgt cagcaaca                                                    18

<210>  62
<211>  20
<212>  DNA
<213>  Artificial Sequence

<220>
<223>  Primer

<400>  62
gggtggcttt gtccagaaca                                                  20

<210>  63

<211>  25
<212>  DNA
<213>  Artificial Sequence

<220>
<223>  Primer

<400>  63
tcgaaagtgg atcttttacg tgttt                                          25


<210>  64
<211>  20
<212>  DNA
<213>  Artificial Sequence

<220>
<223>  Primer

<400>  64
tgacaatgct ccgagcttca                                                20


<210>  65
<211>  18
<212>  DNA
<213>  Artificial Sequence

<220>
<223>  Primer

<400>  65
tgggacgcca ggtggtaa                                                  18


<210>  66
<211>  20
<212>  DNA
<213>  Artificial Sequence

<220>
<223>  Primer

<400>  66
ctgacgttcc atggcatttg                                                20

## Description

The present invention relates to an inhibitor of glycogen synthase kinase 3 (GSK-3) for use in a method of treating or preventing emphysema, a method of activating an emphysema cell, an activated emphysema cell obtained by this method for use in a method of treating or preventing emphysema, and a method of screening for a substance for treating or preventing emphysema.

Chronic obstructive pulmonary disease (COPD) is an emerging serious global health problem, expected to become the third leading cause of death globally 2020 (1). Due to the frequency and poor outcome, COPD is associated with tremendous socioeconomic and individual burden (2). Currently, no causal therapy for COPD is available and avoidance of the major risk factor cigarette smoke exposure is the only effective strategy (3, 4). Importantly, however, COPD also develop in never smokers (5) and has been associated with a variety of endogenous and exogenous factors (6, 7). Among others, developmentally active signaling pathways, such as TGF-β, have been associated with COPD (8-11).

Emphysema is a main feature of COPD, characterised by alveolar airspace enlargement and parenchymal tissue destruction (12, 13). Lung tissue maintenance is believed to be a balanced active process of lung injury and repair, which is impaired in COPD (14, 15). Recent evidence revealed that the WNT/β-catenin pathway, which is essential for lung morphogenesis, is linked with parenchymal lung diseases, particularly with cancer and pulmonary fibrosis (16-18). WNT/β-catenin signaling involves WNT ligand binding to cell surface receptors and cytosolic stabilization and nuclear translocation of β-catenin for target gene expression (19, 20). In the absence of active WNT ligands, β-catenin is constitutively phosphorylated by its interaction with axin, adenomatosis polyposis coli (APC), and glycogen synthase kinase (GSK)-β (called "beta-catenin destruction complex"), and subsequently degraded. In the presence of WNT ligands, two distinct membrane receptors, the frizzled (FZD) or the low density lipoprotein receptor-related proteins (LRP) 5 and 6, are activated upon ligand binding. WNT stimulation leads to phosphorylation of LRP6, which leads the disruption of the β-catenin destruction complex. Cytosolic β-catenin accumulates and undergoes nuclear translocation, where it regulates target gene expression through interaction with members of the T-cell-specific transcription factor/lymphoid enhancer-binding factor (TCF/LEF) family.

Importantly, WNT/β-catenin signaling is involved in lung epithelial injury and repair processes. Canonical WNT/β-catenin activation has been demonstrated to lead to increased proliferation of alveolar epithelial cells, in concert with upregulation of WNT target genes, suggesting a role of active WNT signaling in epithelial cell repair mechanism *in vitro* and *in vivo* (18, 21). Furthermore, inhibition of the WNT target gene WISP1 led to decreased alveolar epithelial proliferation and attenuation of experimental lung fibrosis (18). The impact of WNT/β-catenin signaling on the pathogenesis of COPD, however, has not been clarified thus far. Tian and coworkers suggested that cigarette smoke, intimately related with the development of COPD, would lead to an initial decrease GSK-3β expression, increase inactive phosphorylated GSK3-β and activate β-catenin signaling (49), while others suggested that the WNT pathway needs to be activated in disease-damaged lung tissue (50). Still others suggested that tretinoin (an anti-acne drug commercially available as Retin-A) derived from vitamin A may reverse the effects of emphysema in mice by returning elasticity (and regenerating lung tissue through gene mediation) to the alveoli. However, vitamin A consumption was not known to be an effective treatment or prevention options for the COPD and a follow-up study done in 2010 found inconclusive results ("no definitive clinical benefits") using Vitamin A (retinoic acid) in treatment of emphysema in humans and stated that further research is needed to reach conclusions on this treatment (51).

In summary, COPD is a devastating and poorly understood disease. Currently, no causal therapy for COPD and/or emphysema is available.

Accordingly, it was an object of the present invention to elucidate biological processes involved in human emphysema and the potential of their components as a preventive and therapeutic target in emphysema. For this, the expression, localization and activity of molecular targets were assessed in human emphysematous COPD samples (GOLD stage IV) using quantitative RT-PCR, immunohistochemistry, and western blotting. Targets identified were assayed *in vivo* for their potential use in prophylactic and therapeutic methods in an elastase-induced emphysema model (mice). Surprisingly, we found that main WNT/β-catenin signaling components were largely expressed in the alveolar epithelium in human lung tissue derived from COPD patients, but neither differentially regulation nor active signaling was detected. Further, WNT/β-catenin signaling was downregulated in experimental emphysema, suggesting silenced WNT/β-catenin signaling during the development of emphysematous COPD. Importantly, preventive as well as therapeutic activation of WNT/β-catenin signaling by GSK-3 inhibiton (using Lithium Chloride (LiC1)) led to an attenuation of experimental emphysema, as measured by decreased airspace enlargement, reduced collagen content, and elevated expression of alveolar epithelial cell marker upon WNT activation, thus proving that GSK-3 inhibitors are suitable in the prophylactic and therapeutic treatment of emphysema.

Accordingly, in a first aspect the present invention relates to an inhibitor of glycogen synthase kinase 3 (GSK-3) for use in a method of treating or preventing emphysema.

Emphysema is a long-term, progressive disease of the lung that primarily causes shortness of breath. In people suffering from emphysema, the lung tissues necessary to support the physical shape and function of the lung are destroyed. It is included in a group of diseases called chronic obstructive pulmonary disease (COPD). Emphysema is called an obstructive lung disease because the destruction of lung tissue around bronchioles, makes these airways unable to hold their functional shape upon exhalation. Emphysema is characterized by parenchymal tissue destruction, which is believed to results from imbalanced lung injury and repair processed. However, the pathomechanism leading to increased tissue destruction along with a reduced repair capability of the lung are not fully understood.

The primary cause of emphysema is the smoking of cigarettes. In some cases it may be due to genetic cause, such as the hereditary contribution by alpha 1-antitrypsin deficiency (A1AD).

In normal breathing, air is drawn in through the bronchi and into the alveoli, which are tiny sacs surrounded by capillaries. Alveoli absorb oxygen and then transfer it into the blood. When toxicants, such as cigarette smoke, are breathed into the lungs, the harmful particles become trapped in the alveoli, causing a localized inflammatory response. Compounds released during the inflammatory response (e.g., elastase) can cause the alveolar septum to disintegrate. This condition, known as septal rupture, leads to significant deformation of the lung architecture. These deformations result in a large decrease of alveoli surface area used for gas exchange. This results in a decreased Transfer Factor of the Lung for Carbon Monoxide (TLCO). To accommodate the decreased surface area, thoracic cage expansion (barrel chest) and diaphragm contraction (flattening) take place. Expiration increasingly depends on the thoracic cage and abdominal muscle action, particularly in the end expiratory phase. Due to decreased ventilation, the ability to exude carbon dioxide is significantly impaired. In the more serious cases, oxygen uptake is also impaired. As the alveoli continue to break down, hyperventilation is unable to compensate for the progressively shrinking surface area, and the body is not able to maintain high enough oxygen levels in the blood. The body's last resort is vasoconstricting appropriate vessels. This leads to pulmonary hypertension, which places increased strain on the right side of the heart, the side responsible for pumping deoxygenated blood to the lungs. The heart muscle thickens in order to pump more blood. This condition is often accompanied by the appearance of jugular venous distension. Eventually, as the heart continues to fail, it becomes larger and blood backs up in the liver (called "cor pulmonale").

Treatment or treating is the attempted remediation of a health problem, usually following a diagnosis. A treatment treats a problem, and may lead to its cure, but treatments often ameliorate a problem only for as long as the treatment is continued, especially in chronic diseases. Cures are a subset of treatments that reverse illnesses completely or end medical problems permanently. Prevention or preventing is a way to avoid an injury, sickness, or disease in the first place, and generally it will not help someone who is already ill (though there are exceptions). A treatment or cure is applied after a medical problem has already started, whereas prevention is applied before the medical problem is detectable. The treatment or prevention may be in any subject, particularly a mammal such as cat, dog, rat, mouse, cow, horse, rabbit, or primate, especially in a human.

Glycogen synthase kinase 3 (GSK-3) is a serine/threonine protein kinase, that mediates the addition of phosphate molecules on certain serine and threonine amino acids in particular cellular substrates. The phosphorylation of these other proteins by GSK-3 usually inhibits the target protein (as in the case of glycogen synthase and NFAT). In mammals, GSK-3 is encoded by two known genes GSK-3α and GSK-3β. In a preferred embodiment of the present invention the inhibitor is an inhibitor of glycogen synthase kinase 3β (GSK-3β), which means that the inhibitor preferentially inhibits GSK-3β, i.e. it inhibits GSK-3β rather than GSK-3α.

As mentioned, GSK-3 is known for phosphorylating and thus inactivating glycogen synthase. However, GSK-3 works in the WNT signalling pathway to phosphorylate β-catenin. Phosphorylation leads to ubiquitination and degradation by cellular proteases, preventing it from entering the nucleus and activating transcription factors. When a protein called Disheveled is activated by WNT signaling, GSK-3 is inactivated, allowing β-catenin to accumulate and effect transcription of WNT target genes. GSK-3 also phosphorylates Ci in the Hedgehog (Hh) pathway, targeting it for proteolysis to an inactive form.

GSK-3 is unusual among the kinases in that it usually requires a "priming kinase" to first phosphorylate a substrate, and then GSK-3 additionally phosphorylates the substrate. The consequence of GSK-3 phosphorylation is usually inhibition of the substrate. For example, when GSK-3 phosphorylates another of its substrates, the NFAT family of transcription factors, these transcription factors can not translocate to the nucleus and are therefore inhibited.

GSK-3 can be inhibited by AKT phosphorylation, which is part of insulin signal transduction. Therefore, AKT is an activator of many of the signaling pathways blocked by GSK-3. For example, in the setting of induced AKT signaling, it can be shown that NFAT is dephosphorylated.

It has been shown that the activity of GSK can specifically be inhibited by substances referred to herein as "inhibitor of GSK-3" or "GSK-3 inhibitor". As detailed above, the inhibition of GSK-3 leads to activation of the WNT signaling pathway.

An enzyme inhibitor is a substance that binds an enzyme and decreases its activity. Accordingly, an inhibitor of GSK-3 binds to GSK-3 and decreases its kinase activity. The binding of an inhibitor hinders a substrate from entering the enzyme's active site and/or prevent the enzyme from catalysing its reaction. Inhibitor binding is either reversible or irreversible. Irreversible inhibitors usually react with the enzyme and change it chemically. These inhibitors modify key amino acid residues needed for enzymatic activity. In contrast, reversible inhibitors bind non-covalently and different types of inhibition are produced depending on whether these inhibitors bind the enzyme, the enzyme-substrate complex, or both.

A large number of drugs are enzyme inhibitors, so their discovery and improvement is an active area of research in biochemistry and pharmacology. An enzyme inhibitor used as pharmaceutical is often judged by its specificity (its lack of binding to other proteins) and its potency (its dissociation constant, which indicates the concentration needed to inhibit the enzyme). A high specificity and potency are desirable in order to minimize side effects and toxicity.

The inhibitor of GSK-3 can be a full or partial inhibitor. A full inhibitor is capable of completely blocking the enzyme's activity (100 % inhibition) at a suitable concentration, whereas a partial inhibitor may inhibit the enzyme's activity to a certain extend (e.g. 60 % inhibition), but not to 100 %. However, inhibition in the context of the present invention is based on a specific interaction between the inhibitor and GSK-3 and not unspecific mechanisms (e.g. denaturation of the enzyme).

For administration the GSK-3 inhibitor should be in a pharmaceutical dosage form in general consisting of a mixture of ingredients known to a skilled person in the pharmacotechnical arts such as pharmaceutically acceptable excipients and/or auxiliaries combined to provide desirable characteristics. Examples of such substances are isotonic saline, Ringer's solution, buffers, medium (e.g. EBM, X vivo 10 and X vivo 15) organic or inorganic acids and bases as well as their salts and buffer solutions, sodium chloride, sodium hydrogencarbonate, sodium citrate or dicalcium phosphate, glycols, such a propylene glycol, sugars such as glucose, sucrose and lactose, starches such as corn starch and potato starch, albumins, organic solvents, complexing agents such as citrates and urea, stabilizers, such as protease or nuclease inhibitors. The physiological buffer solution preferably has a pH of approx. 6.0-8.0, especially a pH of approx. 6.8-7.8, in particular a pH of approx. 7.4, and/or an osmolarity of approx. 200-400 milliosmol/liter, preferably of approx. 290-310 milliosmol/liter. The pH of the pharmaceutical composition is in general adjusted using a suitable organic or inorganic buffer, such as, for example, preferably using a phosphate buffer, tris buffer (tris(hydroxyl-methyl)ami-nomethane).

The GSK-3 inhibitor can be administered to a subject by any route suitable for the administration of viable cells. Examples of such routes are intravascularly, intracranially, intracerebrally, intramuscularly, intradermally, intravenously, intraocularly, intraperitoneally, orthotopically in an injured organ or by open surgical procedure. The pharmaceutical may be administered to the subject by e.g. injection, infusion or implantation. It may be administered orthotopically, directly to the tissue or organ to be treated or reconstituted, i.e. the target tissue or target organ, or to a distant site. Most preferably, the medicament comprising the GSK-3 inhibitor (also referred to as pharmaceutical composition) is administered directly to the lung, e.g. by inhalation or local instillation by bronchoscopy.

In another preferred embodiment the inhibitor is a low-molecular-weight chemical compound having a molecular weight of at most 2000 Da, preferably at most 1500 Da, more preferably at most 1000 Da, especially at most 800 Da.

In the fields of pharmacology and biochemistry, a small molecule or low-molecular-weight organic compound is by definition not a polymer. The term small molecule, especially within the field of pharmacology, is usually restricted to a molecule that also binds with high affinity to a biopolymer such as protein, nucleic acid, or polysaccharide and in addition alters the activity or function of the biopolymer. The upper molecular weight limit for a small molecule is approximately 2000 Daltons, preferably at most 1500 Da, more preferably at most 1000 Da, especially at most 800 Da 800, which allows for the possibility to rapidly diffuse across cell membranes so that they can reach intracellular sites of action. In addition, this molecular weight cutoff is a necessary but insufficient condition for oral bioavailability.

Biopolymers such as nucleic acids, proteins, and polysaccharides are not small molecules, although their constituent monomers - ribo- or deoxyribonucleotides, amino acids, and monosaccharides, respectively. Very small oligomers are also usually considered small molecules, such as dinucleotides, peptides such as the antioxidant glutathione, and disaccharides such as sucrose. In a preferred embodiment the low-molecular-weight organic compound is not a small oligomer, particularly not a nucleic acid, peptide or protein.

A well-known inhibitor of GSK-3 is lithium, e.g. as lithium chloride. The mode of inhibition is through competition for Mg²⁺. The bivalent form of zinc, which mimics insulin action, has also been shown to inhibit GSK-3. In vitro, another metal ion, beryllium, inhibits GSK-3. Several new GSK-3 inhibitors have recently been developed, most of which are ATP competitive.

Examples of inhibitors of GSK-3 include - without limitation - aloisines (such as aloisine A and aloisine B), beryllium, bivalent zinc, hymenialdisine (such as dibromo-hymenialdisine), indirubins (such as 5,5'-dibromo-indirubin), Li⁺, maleimides, in particular macrocyclic bisindolylmaleimidis (such as Ro 31-8220, SB-216763, SB-415286, or 3F8) and muscarinic agonists (such as AF102B and AF150).

Preferred examples of the inhibitor are selected from the group consisting of Li⁺, BIO ((2'Z,3'E)-6-Bromoindirubin-3'-oxime; catalog No: B1686, Sigma, St. Louis, MI, USA), SB-216763 (3-(2,4-Dichlorophenyl)-4-(1-methyl-1H-indol-3-yl)-1H-pyrrole-2,5-dione; catalog No: S3442, Sigma, St. Louis, MI, USA), SB-415286 (3-[(3-Chloro-4-hydroxyphenyl)amino]-4-(2-nitrophenyl)-1H-pyrrol-2,5-dione; catalog No: S3567, Sigma, St. Louis, MI, USA). Even more preferred examples of inhibitors of GSK-3 include lithium such as lithium chloride (LiCl), 6-bromoindirubin-3'-oxime (inhibitor IX, (2'*Z*,3'*E*)-6-bromoindirubin-3'-oxime, available from Calbiochem, Darmstadt, Germany), inhibitor XII (TWS119) or inhibitor XV (both also available from Calbiochem, Darmstadt, Germany), and/or 3F8 (52) especially lithium such as lithium chloride (LiCI).

Preferably, the inhibitor is for use in a method of preventing emphysema in a subject. As shown in the Examples (cf. II.3), a GSK-33 inhibitor is also useful in the prevention of emphysema. The inventors proved that LiCl administration started on the day of emphysema induction was preventive for the development of emphysema *in vivo.* The analysis of key features of emphysema, such as destroyed lung architecture and surface, decreased alveolar epithelial type II (ATII) cells, or enhanced collagen deposition revealed marked attenuated emphysematous changes. Further, increased appearance of SP-C-positive cells with enhanced staining of the WNT/β-catenin signaling component WNT3a, nuclear β-catenin staining, increased SP-C protein expression, improved alveolar structure, increased airway surface area. In summary, preventive WNT/β-catenin activation led to an attenuation of emphysema, as measured by decreased airspace enlargement, gas exchange area, and improved alveolar structure upon WNT activation.

Prevention or preventing refers to measures taken to prevent diseases, rather than curing them or treating their symptoms. The term contrasts in method with curative and palliative medicine. In the context of the present invention prevention of emphysema means that the development of emphysema is prevented before any signs or symptoms of the disease are detectable, i.e. at a stage in which the subject is still healthy with respect to emphysema.

The preventive use of the inhibitor is of particular advantage in subjects being at risk of developing an emphysema. There are several reasons because of which the subject might be at risk of developing an emphysema. Particularly, the subjects may exhibit an increased risk of developing an emphysema due to (first and/or second hand) smoke, alpha 1-antitrypsin deficiency, exposure to occupational chemicals or dust (such as miners or women who cook over open fires), indoor and outdoor air pollution, abnormal lung development (e.g. prenatally), familial disposition, connective tissue disorders, or immune-related diseases, such as HIV.

Though emphysema is caused by different types of environmental pollution, tobacco smoke is by far the most potent cause. Most importantly, both, first and second hand smoke, lead to enhanced risk of developing an emphysema. Tobacco smoke contains over 4000 chemicals. These chemicals are thought to damage the alveoli and ultimately break them down. The resulting larger sacks are less efficient in processing the inhaling and exhaling of oxygen and carbon dioxide respectively. This makes our breathing difficult. The larger sacks are responsible for trapping air and making our breathing difficult. The end result is severe breathlessness.

Besides smokers, also patients with alpha 1-antitrypsin deficiency (A1AD) are more likely to suffer from emphysema. A1AD allows inflammatory enzymes (such as elastase) to destroy the alveolar tissue. Most A1AD patients do not develop clinically significant emphysema, but smoking and severely decreased A1AT levels (10-15%) can cause emphysema at a young age. The type of emphysema caused by A1AD is known as *panacinar* emphysema (involving the entire acinus) as opposed to *centrilobular* emphysema, which is caused by smoking. Panacinar emphysema typically affects the lower lungs, while centrilobular emphysema affects the upper lungs. A1AD causes about 2% of all emphysema. Smokers with A1AD are at the greatest risk for emphysema. Mild emphysema can often develop into a severe case over a short period of time (1-2 weeks).

In particular with respect to an increasing global health problem, exposure to occupational dust and chemicals has been shown to be associated with the development of emphysema. Accordingly subject with increased exposure to occupational dust (such as miners, roadmen, office workers (exposure to toner), or women who cook over open fires, as outlined in detail above) could benefit from a preventive emphysema therapy.

Finally, also subjects with a familial disposition of emphysema or handicapped by abnormal lung development in the womb are more likely to develop the disease. Accordingly, they would also benefit from a preventive emphysema therapy.

This is also important for people affected by other disease, such as connective tissue disorders, or immune-related diseases, such as HIV, as these people exhibit an increased risk of developing an emphysema.
In a second aspect, the present invention relates to a method of activating an emphysema cell, the method comprising
- contacting the cell with a GSK-3 inhibitor, thereby activating the cell.

In patent systems, in which the above method would be regarded as non patentable, if carried out *in vivo,* the method is limited to an *in vitro* method.

In the context of the second aspect of the present invention, the GSK-3 inhibitor may be defined as described above in the context of the first aspect of the present invention.

As detailed above, lung tissue maintenance (balanced active process of lung injury and repair) is believed to be impaired in emphysema lung cells. Particularly, it has been shown that repair process is disturbed leading to reduced cell repair. Administration of GSK-3 inhibitor led to increased lung repair in emphysema by activation of emphysema cells.

Activation is this context means that lung repair capability of an emphysema cell is increased in the presence of GSK-3 inhibitor relative to an emphysema cell not contacted with the inhibitor.

The emphysema cell may be any lung cell derived from an emphysema, particularly from the lung parenchyma, especially from the alveolar epithelium. The alveolar epithelium is composed of alveolar type I (ATI) and type II (ATII) cells. ATII cells supposedly serve as progenitors with "stem cell like character", initiating alveolar epithelial restoration, with ATII cells either giving rise to new ATII or differentiating into ATI cells. The ability of these cells to undergo repair underlines the high degree of plasticity. Moreover, ATII and ATI cells produce and secrete components of the extracellular matrix and growth factors thereof, which facilitates restoration of the interstitium and, subsequently, functional alveolar structure (53).

Particularly, the emphysema cell is an ATII or ATI cell. Further, targeted cells would be an interstitial cell, such as a fibroblast, which has close interaction to the epithelium, and a functional epithelial-fibroblasts interaction is important for repair.

In a third aspect of the invention the activated emphysema cell obtained by a method the invention may be used in a method of treating or preventing emphysema in a subject.

In the context of the third aspect of the present invention, the features "activated emphysema cell", "treating", "preventing", "subject" and/or "emphysema" may be defined as described above in the context of the first and second aspect of the present invention.

Accordingly, the cells may be used as a medicament, which may optionally encompass excipients and/or auxiliaries. Further details on medication are given above. It should be noted that a sufficient amount of cells should be present in the medicament. It is also possible to combine the cells of two or more subjects into one medicament.

In a preferred embodiment the number of cells to be administered to a subject amounts to at least 1 Mio, more preferable at least 2 Mio, still more preferably at least 10 Mio MAB-like cells per treatment. It might be necessary to administer the cells in several doses, e.g. on different days for successful treatment.

In the context of the present invention, cells have been obtained from a subject, e.g. by isolation of primary epithelial cells or fibroblasts out of tissue obtained by biopsies or brushing procedures during bronchoscopy.

These cells are activated by incubation with GSK-3 inhibitor. The cells may be kept in cell culture at conditions and for a time allowing for activation of the cells and optionally for cell propagation. Suitable cell culture conditions are well known to the skilled person. Exemplary conditions are for example that cells phenotypically characterized and cultured in DMEM plus 10% FCS, 2 mM 1-glutamine, 100 U/ml penicillin, and 100g/ml streptomycin and cultured in a humidified atmosphere of 5% CO2 at 37°C. After treatment cells would be further characterized and analysed using several functional methods, such as thymidine incorporation, migration (Boyden chamber) and apoptosis (TUNEL) assays, and immunofluorescenct live cell imaging.

Thereafter, the cells are collected and administered to a subject in order to treat or prevent emphysema in said subject. The administration may be for example by inhalation or local instillation by bronchoscopy.

Though the present invention is not limited thereto, the cell is preferably an autologous cell.

Autologous in the context of transplantation of organs, tissues or even cells means that the transplant donor and recipient are the same individual. A transplant by such autologous procedure is referred to as an autograft or autotransplant. It is contrasted with xenotransplantation (from other species) and allotransplantation (from other individual of same species).

In a preferred embodiment the subject receiving the activated cell may be a subject being at risk of developing an emphysema due to smoking, alpha 1-antitrypsin deficiency, exposure to occupational dust or chemicals, familial disposition, or other disorders, as detailed above. In this embodiment the transplanted cell may by a lung cell at risk of developing into en emphysema.

In a forth aspect, the present invention relates to a method of screening for substance for treating or preventing emphysema, the method comprising
- contacting a GSK-3 or functionally active derivative thereof with a compound; and
- determining the activity of the GSK-3 in the presence of the compound,
wherein the compound is identified as a substance for of treating or preventing emphysema, if the activity of GSK-3 in the presence of the compound is reduced relative to a control.

In the context of the fourth aspect of the present invention, the features "GSK-3", "treating", "preventing", and/or "emphysema" may be as defined above.

Preferably, the GSK-3 is GSK-3β, as defined above.

As detailed above, glycogen synthase kinase 3 (GSK-3) is a serine/threonine kinase that was first isolated and purified as an enzyme capable of phosphorylating and inactivating the enzyme glycogen synthase. Beyond its role in glycogen metabolism, GSK-3 acts as a downstream regulatory switch that determines the output of numerous signalling pathways initiated by diverse stimuli. The pathways in which GSK-3 acts as a key regulator, when dysregulated, have been implicated in the development of human diseases such as diabetes, Alzheimer's disease, bipolar disorder and cancer. Given its involvement in many pathophysiological processes and diseases, GSK-3 is a tempting therapeutic target. In the context of emphysema inhibitors of GSK-3 are of particular interest.

The claimed screening method includes contacting a GSK-3 or functionally active derivative thereof with a compound. There are two mammalian GSK-3 isoforms encoded by distinct genes: GSK-3α and GSK-3β. GSK-3α has a mass of 51 kDa, whereas GSK-3β is a protein of 47 kDa. The difference in size is due to a glycine-rich extension at the N-terminus of GSK-3α. Although highly homologous within their kinase domains (98% identity), the two gene products share only 36% identity in the last 76 C-terminal residues. Homologues of GSK-3 exist in all eukaryotes examined to date and display a high degree of homology; isoforms from species as distant as flies and humans display >90% sequence similarity within the kinase domain.

The amino acid sequence of the human GSK-3β consists of 420 amino acids and is available at PubMed under the accession no. NP_001139628.1. The amino acid sequence of the human GSK-3α consists of 483 amino acids and is available at PubMed under the accession no. NP_063937.2. However, GSK-3 may be also derived form any other species and the sequence of GSK-3 proteins of other species has already been published. Examples include *Mus musculus, Rattus norwegicus, Bos taurus, Equus caballus, Canis lupus familiaris, Danio rerio, Pan troglodytes, Gallus gallus, Ovis aries, Macaca mulatta, Sus scrofa,* and *Drosophila melanogaster.*

In addition to any natural occurring GSK-3 variant, such as a species variant or splice variant, modified GSK-3 proteins may be also used. It should be noted that the modified GSK-3 protein or GSK-3 variant is a functionally active variant, in that the variant maintains its biological function as a serine/threonine kinase. Preferably, maintenance of biological function, e.g. regulation of WNT pathway, is defined as having at least 50 %, preferably at least 60 %, more preferably at least 70 %, 80 % or 90 %, still more preferably 95 % of the enzyme activity of the natural occurring GSK-3. The biological activity may be determined as described in the examples.

The variant may be a molecule having a domain composed of a naturally occurring GSK-3 and at least one further component. For example, the protein may be coupled to a marker, such as a tag used for purification purposes (e.g. 6 His (or HexaHis) tag, Strep tag, HA tag, c-myc tag or glutathione S-transferase (GST) tag). If a e.g. highly purified HEBP1 protein or variant should be required, double or multiple markers (e.g. combinations of the above markers or tags) may be used. In this case the proteins are purified in two or more separate chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag), the 6xHis-tag100-tag (6 histidine residues fused to a 12-amino-acid protein of mammalian MAP-kinase 2), 8xHis-HA-tag (8 histidine residues fused to a haemagglutinin-epitope-tag), His-MBP (His-tag fused to a maltose-binding protein, FLAG-HA-tag (FLAG-tag fused to a hemagglutinin-epitope-tag), and the FLAG-Strep-tag. The marker could be used in order to detect the tagged protein, wherein specific antibodies could be used. Suitable antibodies include anti-HA (such as 12CA5 or 3F10), anti-6 His, anti-c-myc and anti-GST. Furthermore, the HEBP1 protein could be linked to a marker of a different category, such as a fluorescence marker or a radioactive marker, which allows for the detection of HEBP 1. In a further embodiment, HEBP1 could be part of a fusion protein, wherein the second part could be used for detection, such as a protein component having enzymatic activity.

In another embodiment of the present invention, the GSK-3 variant could be a GSK-3 fragment, wherein the fragment is still functionally active. This may include GSK-3 proteins with short C- and/or N-terminal deletions (e.g. deletions of at most 20, 19, 18, 17, 16, 15, 14, 13, 12, 11, 10, 9, 8, 7, 6 5, 4, 3, 2, or 1 amino acid). Additionally, the GSK-3 fragment may be further modified as detailed above for the GSK-3 protein.

Alternatively or additionally, the GSK-3 protein or variant thereof as described above may comprise one or more amino acid substitution(s), particularly in regions not involved in the enzymatic reaction. However, conservative amino acid substitutions, wherein an amino acid is substituted with a chemically related amino acid are preferred. Typical conservative substitutions are among the aliphatic amino acids, are among the amino acids having aliphatic hydroxyl side chains, are among the amino acids having acidic residues, among the amid derivates, among the amino acids with basic residues, or the amino acids having aromatic residues. The GSK-3 protein or fragment or variant with substitution may be modified as detailed above for the GSK-3 protein or fragment or variant. In the following description of the invention all details given with respect to GSK-3 protein also relate to functionally active variants thereof, unless stated otherwise.

However, most preferably, the GSK-3 protein is a naturally occurring GSK-3 protein (especially GSK-3β), still more preferably, a naturally occurring human GSK-3 protein (especially GSK-3β).

The contacting and determining may be done in the presence of further elements such as a substance for GSK-3 or means for detecting the activity of GSK-3 in order to identify the compound as an inhibitor of GSK-3. Suitable substrates and means for detection are known to the skilled person.

The compound tested with the method of the present invention may be any test substance or test compound of any chemical nature. It may already be known as a drug or medicament for a disease. Alternatively, it may be a known chemical compound not yet known to have a therapeutic effect in another embodiment and the compound may be a novel or so far unknown chemical compound. The compound may be also a mixture of test substances or test compounds.

In one embodiment of the screening method of the present invention, the compound is provided in form of a chemical compound library. Chemical compound libraries include a plurality of chemical compounds and have been assembled from any of multiple sources, including chemical synthesized molecules or natural products, or have been generated by combinatorial chemistry techniques. They are especially suitable for high-throughput screening and may be comprised of chemical compounds of a particular structure or compounds of a particular organism such as a plant. In the context of the present invention, the chemical compound library is preferably a library comprising proteins and polypeptides or small organic molecules. Preferably a small organic molecule is less than 500 daltons in size, particularly a soluble, non-oligomeric, organic compound.

In the context of the present invention, GSK-3 is contacted with the compound for a time and under conditions suitable for inhibiting GSK-3 and detecting the same. Suitable conditions include appropriate temperature and solution to avoid e.g. denaturation of proteins involved or to maintain viable cells, if present. Suitable conditions will depend from the particular method chosen and the skilled person will be able to select the same based on his general knowledge.

After the contacting of GSK-3 with the compound, the effect of the compound on GSK-3 is detected. In the following, a series of different detection systems will be described in more detail. However, it should be understood that these are exemplary and other test systems may be also appropriate.

If the activity of GSK-3 in the presence of the compound is reduced relative to a control, the compound is identified as a substance for of treating or preventing emphysema. In the context of the present invention, activity of GSK-3 reduced in comparison to a control, if the activity of GSK-3 contacted with the compound is significant lower than that of a control (e.g. the same GSK-3 not contacted with the compound). The person skilled in the art knows statistical procedures to assess whether two values are significantly different from each other such as Student's t-test or chi-square tests.

In a preferred embodiment, the activity of GSK-3 amounts to at most 90 %, preferably at least 80 %, more preferably at most 70 %, 60 %, or 50 %, still more preferably at most 25 % and most preferably at most 10 % of the control.

As detailed above, suitable methods of determining activity of GSK-3 are known to te skilled person and given in the Examples. The determining could be either by determined the activity of GSK-3 directly (e.g. kinase activity) (54, 55) or by detecting the effect on a component in the WNT/β-catenin signaling pathway downstream of GSK-3, e.g. protein or mRNA expression profile of the main WNT/β-catenin signaling components (see Examples).

The invention is not limited to the particular methodology, protocols, and reagents described herein because they may vary. Further, the terminology used herein is for the purpose of describing particular embodiments only and is not intended to limit the scope of the present invention. As used herein and in the appended claims, the singular forms "a," "an," and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the invention. Although any methods and materials similar or equivalent to those described herein can be used in the practice of the present invention, the preferred methods, and materials are described herein.

The invention is further illustrated by the following Figures and Examples, although it will be understood that the Figures and Examples are included merely for purposes of illustration and are not intended to limit the scope of the invention unless otherwise specifically indicated.

### FIGURES

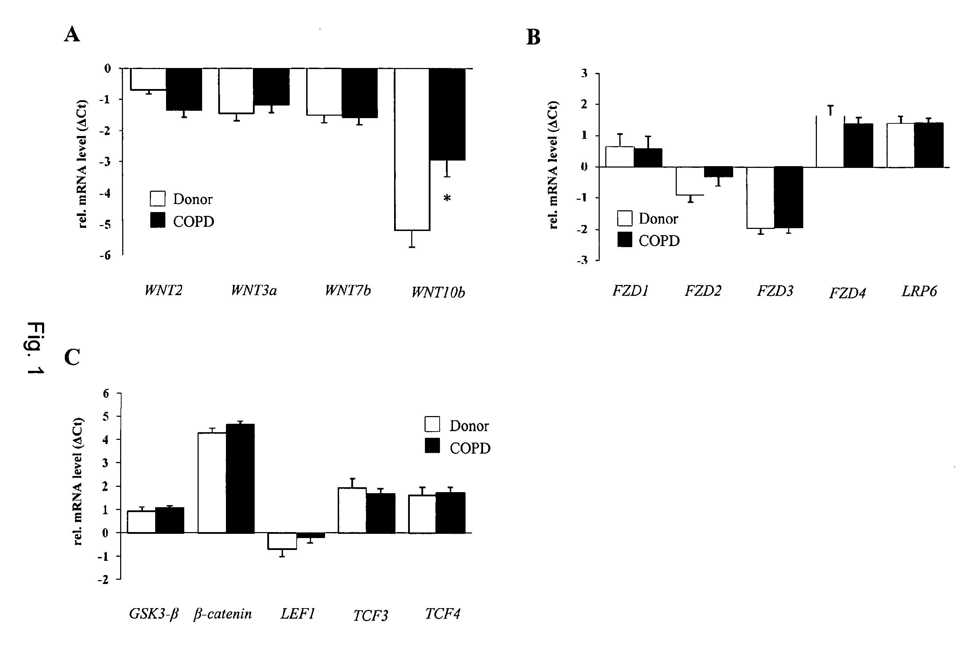

**Figure 1****. The mRNA expression profile of WNT/**β-**catenin signaling components in COPD and transplant donor patients.** The mRNA level of (A) the WNT ligands *WNT2*, *3a, 7b,* and *10b,* (B) the receptors frizzled (*FZD) 1-4,* low density lipoprotein-related protein *(LRP)* 6, and (C) the intracellular signal transducers glycogen synthase kinase *(GSK)-3β, β-catenin,* lymphoid enhancer-binding factor *(LEF) 1*, T-cell-specific transcription factor *(TCF)* 3, *TCF* 4, were assessed in transplant donor and COPD lung specimen by quantitative (q)RT-PCR. Results are derived from 12 donors and 12 COPD patients and presented as mean ±s.e.m., * p< 0.05.

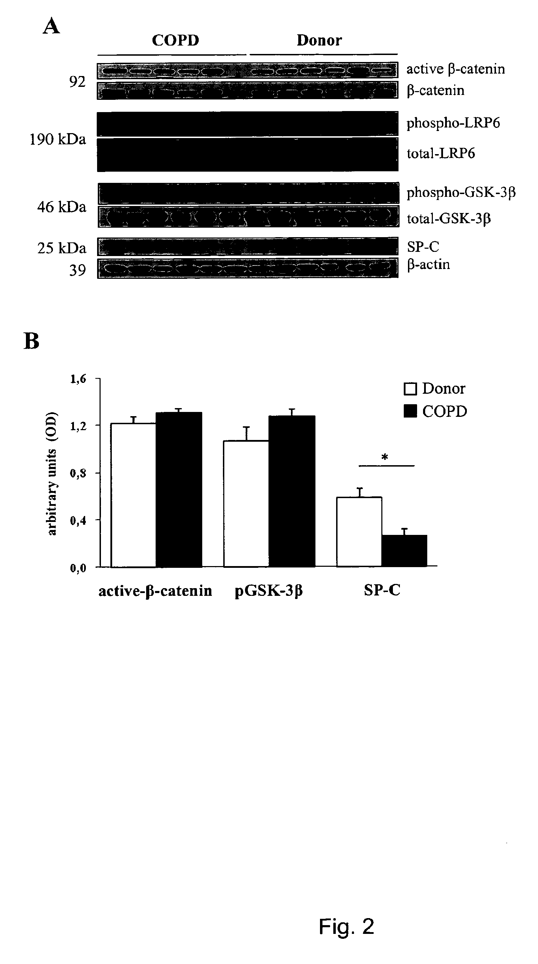

**Figure 2****. Activity of the WNT/**β**-catenin pathway in lung homogenates of COPD and transplant donor patients.** (A) The expression of active WNT components in lung homogenates of transplant donor and COPD patients was analyzed by immunoblotting of phosphorylated and total GSK-3β and low density lipoprotein-related protein (LRP) 6, respectively, as well as active and total β-catenin. Blotting of total β-catenin GSK-3β, LRP6 and β-actin served as loading controls. Immunoblotting of surfactant protein-C (SP-C) was used as a positive control. (B) Results were confirmed by densitometry and presented as mean ±s.e.m., * p,0.05.

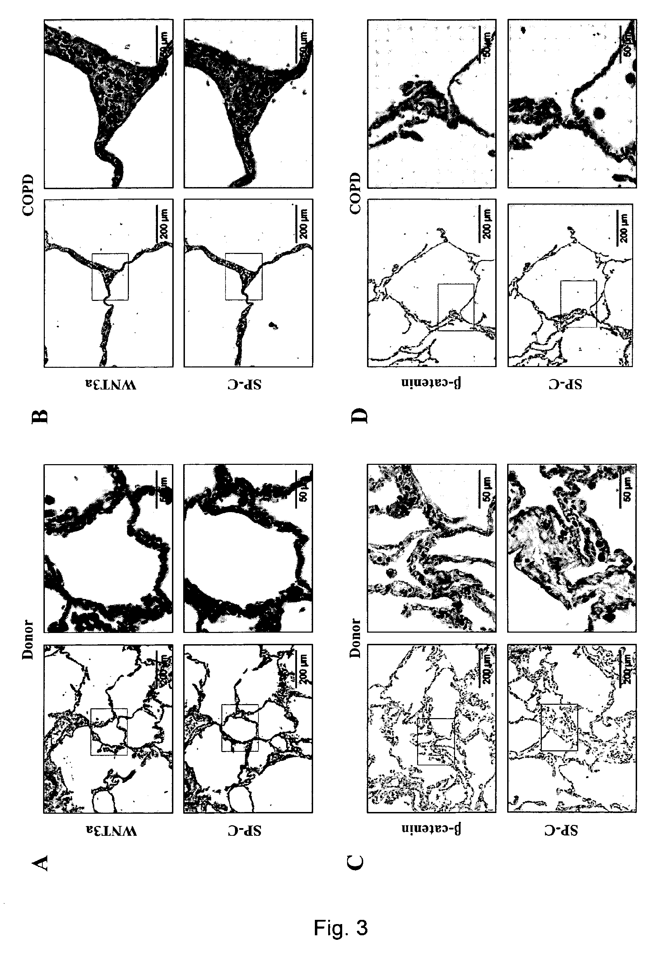

**Figure 3****. Expression and localisation of WNT3a and** β**-catenin in lung tissues of COPD and transplant donor patients.** Immunohistochemical staining was performed on tissue sections derived from transplant donor and COPD lungs, respectively. Serial section with staining of (A-B) SP-C and WNT3a as well as (C-D) SP-C and β-catenin, were used to demonstrate co-localisation of the investigated proteins in alveolar epithelial type II (ATII) cells. Representative pictures of two independent experiments using at least three different transplant donor (A, C) or COPD (B, D) lung tissues, respectively. Magnification is indicated in the pictures.

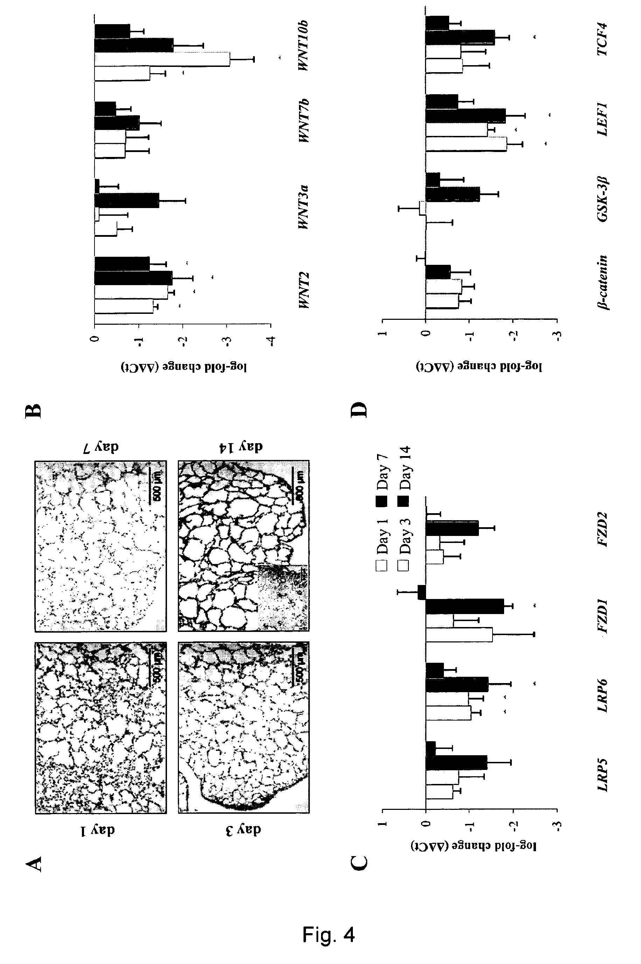

**Figure 4****. The mRNA expression profile of WNT/**β**-catenin signaling components in experimental emphysema in mice.** (A) Histological assessment of lung structure in experimental emphysema. Mice were subjected to elastase instillation, and lungs were obtained on day 1, 3, 7 and 14 after challenge for immunohistochemistry and RNA isolation. Stainings are representative of 2 independent experiments using at least 3 different elastase- or control-treated lung tissues for each time point. The mRNA level of (B) the WNT ligands WNT2, 3a, 7b, and 10b, (C) the receptors *LRP5, 6, FZD1* and 2, and (D) the intracellular signal transducers and transcription factors *GSK-3β, β-catenin, LEF1* and *TCF4* were assessed in mice subjected to elastase after indicated time points (n = 4 each). Results are presented as log-fold change (mean ±s.e.m.), * p<0.05.

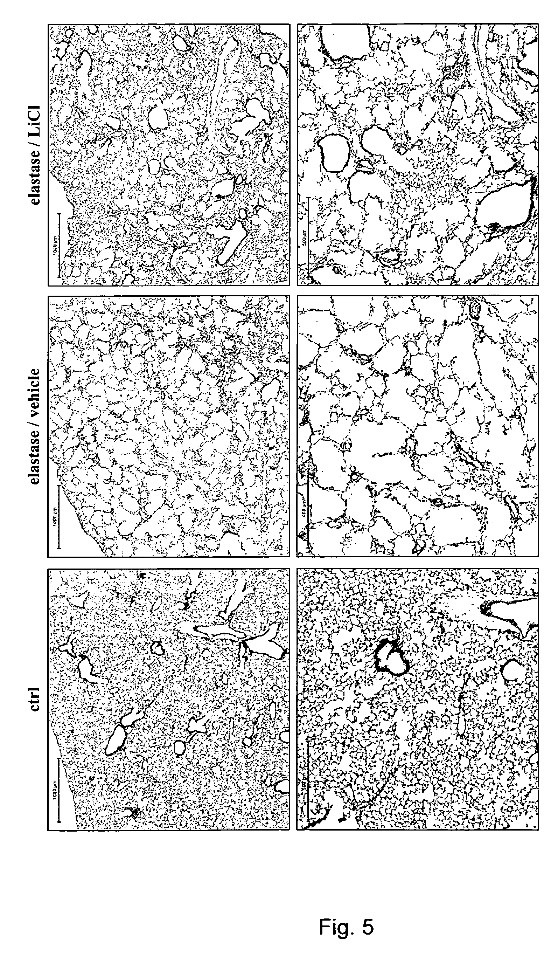

**Figure 5****. Histological assessment of lung structure after preventive WNT/**β**-catenin activation in experimental lung emphysema.** Mice were subjected to elastase instillation, and treated with LiC1, as described before. After seven days, lungs were processed for hematoxylin and eosin staining of lung sections (magnification as indicated). Stainings are representative of two independent experiments using at least three different elastase- or control-treated lung tissues, respectively.

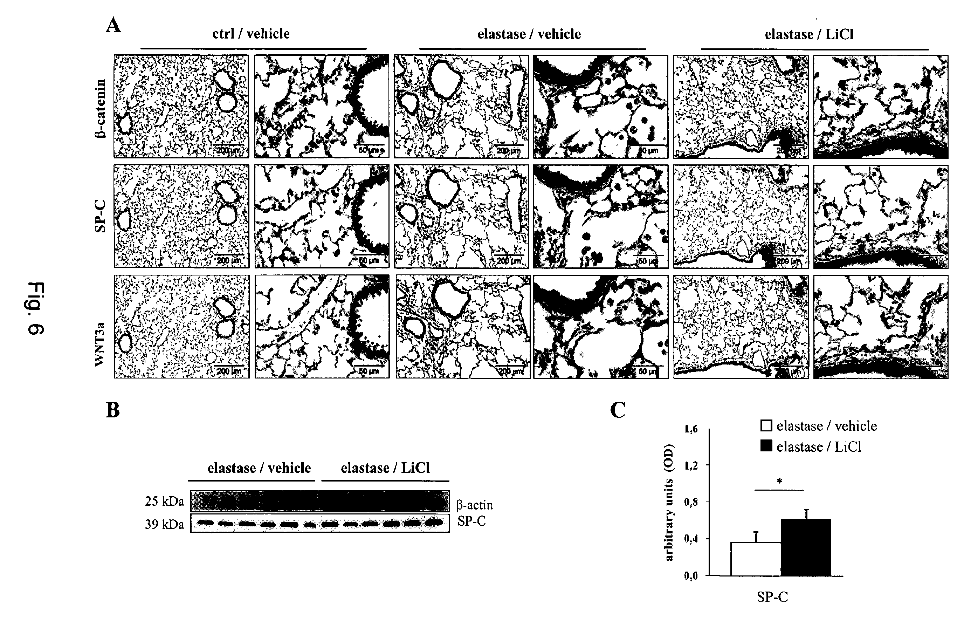

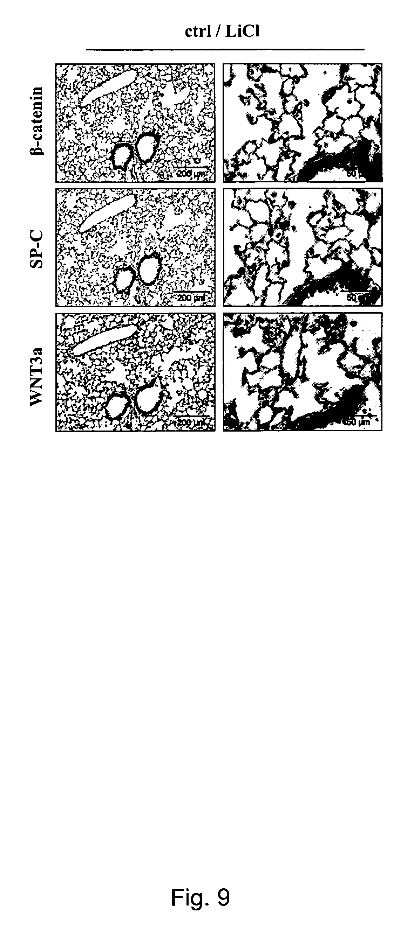

**Figure 6****. Expression of WNT/**β**-catenin signaling components in experimental lung emphysema.** (A) Immunohistochemical staining for β-catenin, SP-C, and WNT3a was performed on whole-lung sections of emphysematous mouse lungs treated with LiC1 or vehicle, as indicated. Stainings of control mice treated with Lithium chloride are presented in Figure 9. Stainings are representative of two independent experiments using at least four different elastase- or control-treated lung tissues, respectively. (B) The expression of SP-C in lung homogenates of emphysematous mice treated with LiC1 compared with control mice, was analyzed by immunoblotting and confirmed by densitometry (C). Results are presented as mean ±s.e.m., * p<0.05.

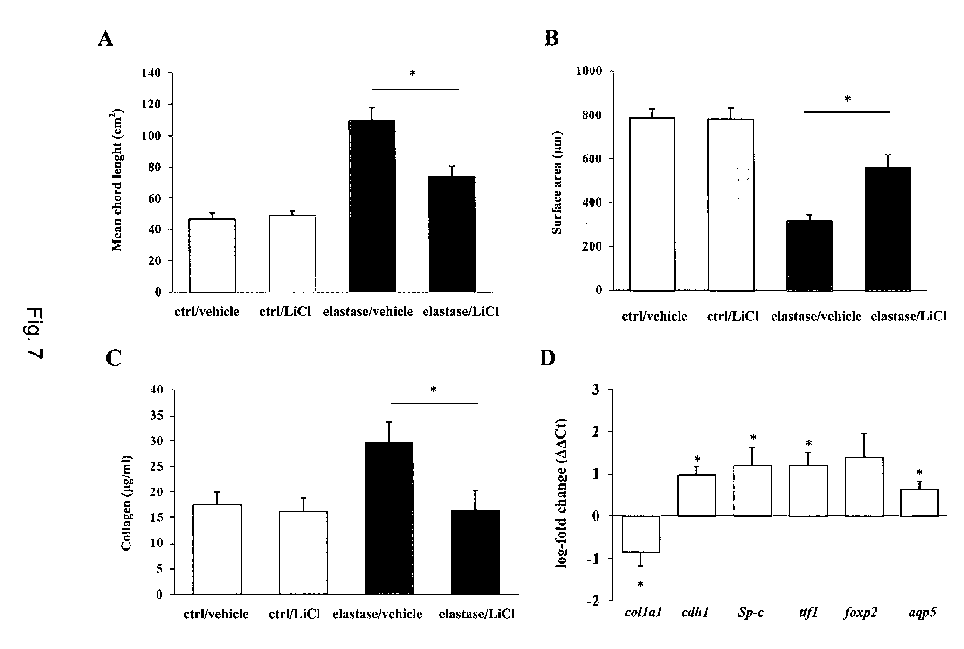

**Figure 7****. Quantitative analysis of lung structure after preventive WNT/**β**-catenin activation in experimental lung emphysema.** The mean chord length (A) and the surface area (B) were assessed by morphometric analysis. (C) The collagen content was measured in total lung homogenates. (D) The mRNA level of the ECM component type I collagen α1 (*collal*), the epithelial cell marker cadherin 1 (*cdh1*), surfactant protein-C *(sp-c),* thyroid transcription factor 1 (*ttf1*), forkhead box P2 (*foxp2*), and aquaporin 5 (*aqp5*) were assessed by qRT-PCR. Results are presented as log-fold change of mRNA level in LiC1-treated versus untreated lungs (mean ±s.e.m.), * p<0.05.

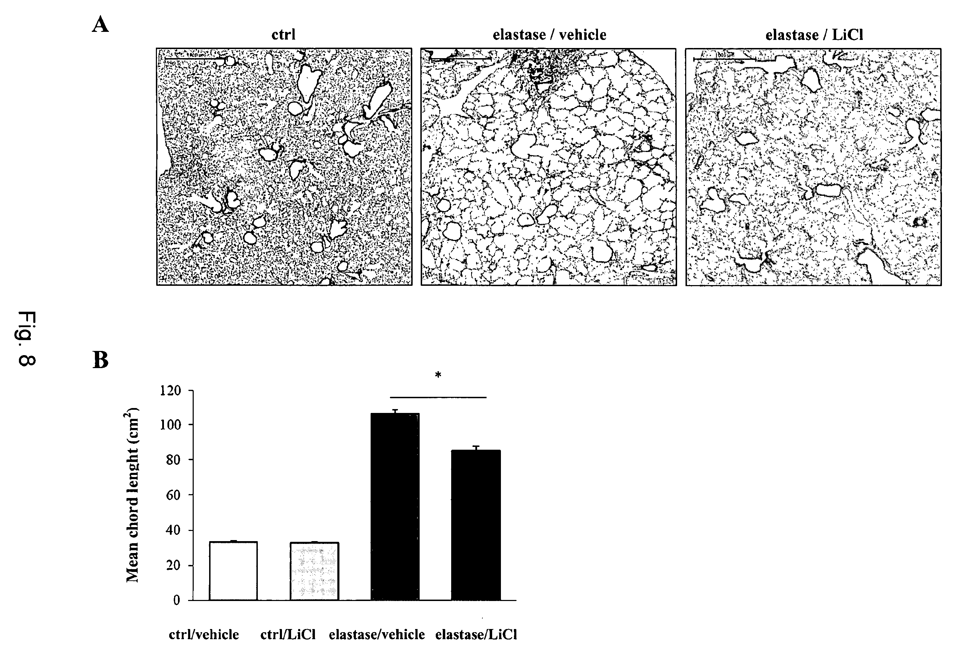

**Figure 8****. Histological assessment of lung structure after therapeutic WNT/**β**-catenin activationin experimental lung emphysema.** (A) Mice were subjected to elastase instillation, and treated with LiC1, as described in Figure 5A. After 14 days, lungs were processed for hematoxylin and eosin staining of lung sections (magnification as indicated). (B) The mean chord length was assessed by morphometric analysis, as described in detail in the supplement of this manuscript. Results are presented as mean ±s.e.m., * p<0.05.
**Figure 9****. Epithelial expression of WNT/**β**-catenin signaling components in healthy mice treated with Lithium Chloride.** Immunohistochemical staining for β-catenin, Surfactant Protein-C (SP-C) and WNT3a was performed on whole-lung sections of healthy mice treated daily with Lithium Chloride (ctrl/LiC1), as indicated (magnification as indicated). Stainings are representative of two independent experiments using at least 3 different elastase- or control-treated lung tissues.

### EXAMPLES

### I. MATERIALS AND METHODS

Antibodies and reagents: The following antibodies were used in this study: Active β-catenin (#05-665), Surfactant Protein C (SP-C) (#AB3786), total β-catenin (#9562), phospho-S9- and total GSK-3β (#9336 and #9315, respectively), phospho- and total LRP6 (#2568 and #2560, respectively; all from Cell Signaling Technology, Beverly, MA), WNT1 (ab15251, Abcam, Cambridge, UK), WNT3a (38-2700, Zymed Laboratories/Invitrogen, Carlsbad, CA), α-smooth muscle actin (SMA, A2547, Sigma-Aldrich, Saint Louis, MO). Lithium chloride, for molecular biology, ≥ 99% (#L9650) and pancreatic elastase from porcine pancreas (#45124) were purchased from Sigma-Aldrich.

Human tissues: Lung tissue was obtained from 12 COPD patients classified as Global Initiative for Chronic Obstructive Lung Disease (GOLD) IV undergoing lung transplant due to their underlying COPD (5 females, 7 males; mean age = 56±5 years; mean FEV1= 16.1±2.8%; mean FEV1/FVC = 43.4±14.6%) and 12 control subjects (transplant donors; 6 females, 6 males; mean age 42±10 years). COPD samples were taken from the parenchyma with histological validation of emphysematous changes. The study protocol was approved by the Ethics Committee of the Justus-Liebig-University School of Medicine. Informed consent was obtained in written form from each subject for the study protocol.

Animals: Six- to eight-week-old pathogen-free female C57BL/6N mice (Charles River Laboratories) were used throughout this study. All experiments were performed in accordance with the guidelines of the Ethics Committee of the University of Giessen School of Medicine and approved by the Regierungspräsidium Giessen, Hesse, Germany. Mice had free access to water and rodent laboratory chow. Pancreatic elastase was dissolved in sterile PBS and applied orotracheally (100U/kg BW). Control mice received 80 µl sterile PBS. Lung tissues were excised and snap-frozen or inflated with 4 % (m/v) paraformaldehyde in PBS (PAA Laboratories) at 21 cm H₂O pressure for histological analyses.

WNT/β-catenin pathway activation *in vivo:* The WNT/β-catenin signaling pathway in lungs from C57BL/6N mice was activated via intraperitoneal injection of lithium chloride (200mg/KG/BW/day) (22). Lithium chloride mimics members of the WNT family of signalling proteins by inhibiting the activity of GSK-3β and thus causes intracellular accumulation of β-catenin, a feature associated with the canonical WNT/β-catenin signalling pathway (23). Under these circumstances, β-catenin can enter the nucleus and influence the expression of target genes. Lithium chloride was diluted in sterile water and fresh stock was prepared for every injection. Mice were treated on a daily basis, from day 0, the day of elastase subjection to day 7 in the preventive regime, and from day 7 to 14 in the therapeutic regime, respectively. The following scheme illustrates preventive and therapeutic activation of WNT/β-catenin signaling in experimental emphysema:

preventive approach    therapeutic approach
PBS 80 µl at day 0 (n=6)    Sterile Water i.p. 600 µl/day or
Lithium Chloride i.p. 200 mg/kg BW/day
Elastase 100 U/kg BW at day 0 (n=6)    Sterile Water i.p. 600 µl/day or
Lithium Chloride i.p. 200 mg/kg BW/day
PBS 80 µl at day 0 (n=6)    No treatment    Sterile Water i.p. 600 µl/day or Lithium
Chloride i.p. 200 mg/kg BW/day
Elastase 100 U/kg BW at day 0 (n=6)
No treatment    Sterile Water i.p. 600 µl/day or Lithium
Chloride i.p. 200 mg/kg BW/day
↑    ↑    ↑
day of subjection    7 days    14 days

Mice were subjected to a single orotracheal application of elastase, which led to the development of pulmonary emphysema from day 1 to 14. WNT/β-catenin activation by treatment with lithium chloride (LiC1) was initiated on day of elastase subjection to day 7 (referred to as *preventive approach),* or from day 7 to day 14 (referred to as *therapeutic approach),* using the indicated concentrations (daily application intraperitonal (i.p.), n = 6 for each group).

Quantitative morphometry: Design-based stereology was used to analyze sections using an Olympus BX51 light microscope equipped with a computer assisted stereological toolbox (CAST-Grid 2.1, Olympus, Denmark), as outlined in the online supplement in more detail and previously described (24).

Statistical analysis: All ΔCt values obtained from real-time RT-PCR were analyzed for normal distribution using the Shapiro-Wilk test, using assignment of a normal distribution with p > 0.05. Normality of data was confirmed using quantile-quantile plots. The means of indicated groups were compared using two-tailed Student's *t*-test, or a one-way analysis of variance (ANOVA) with Tukey HSD post hoc test for studies with more than 2 groups. Results were considered statistically significant when p < 0.05.

Reverse transcription and quantitative RT-PCR: Total RNA was extracted using Qiagen extraction kits according to the manufacturer's protocol, and cDNAs were generated by reverse transcription using SuperScriptTM II (Invitrogen). Quantitative (q)RT-PCR was performed using fluorogenic SYBR Green and the Sequence Detection System Fast 7500 (PE Applied Biosystems). HPRT1 and PBGD, ubiquitously and equally expressed genes free of pseudogenes, were used as a reference gene in all human and mouse qRT-PCR reactions, respectively. PCR was performed using the primers listed in Table 1 and 2, respectively, at a final concentration of 200 nM. Relative transcript abundance of a gene is expressed in ΔCt values (ΔCt =ct^{reference} - Ct^{target}). Relative changes in transcript levels compared to controls are ΔΔCt values (ΔΔCt =ΔCt^{treated} - ΔCt^{control}). All ΔΔCt values correspond approximately to the binary logarithm of the fold change as mentioned in the text. When relative transcript abundance is of information, expression levels are given in ΔCt levels.

Immunohistochemistry: Lungs were placed in 4% (w/v) paraformaldenyde after explantation, and processed for paraffin embedding. Sections (3 µm) were cut, mounted on slides, subjected to antigen retrieval, and quenching of endogenous peroxidase activity using 3% (v/v) H₂O₂ for 20 min. Immune complexes were visualized using suitable peroxidase-coupled secondary antibodies, according to the manufacturer's protocol (Histostain Plus Kit; Zymed/Invitrogen).

Western blot analysis: Human lung tissue specimens were homogenized in extraction buffer [20 mM Tris-Cl, 150 mM NaCl, 1 mM EDTA, 1 mM EGTA, 1% (v/v) Triton X-100, supplemented with Complete^{™} Proteinase Inhibitor Cocktail (Merck Biosciences)] and whole proteins were extracted by centrifugation (12,000 x g) for 10 mm at 4°C. Samples containing 25 mg of protein were separated by electrophoresis on a 10% SDS-polyacrylamide gels. The separated proteins were transferred to nitrocellulose membranes (Invitrogen), blocked with 5% skim milk, and incubated with the indicated antibodies. Proteins were then visualized by enhanced chemiluminescence detection (ECL, Amersham Biosciences, Uppsala, Sweden), as reported. Prior to reprobing, nitrocellulose membranes were incubated with stripping stripping buffer [100 mM 2-mercaptoethanol, 2% SDS, and 62.5 mM Tris-HCl (pH 6.7)] at 50°C for 30 min.

Collagen assay: Whole-mouse lung homogenates were used for in vivo analysis. Total collagen content was determined using the Sircol Collagen Assay kit (Biocolor). Equal amounts of protein lysates were added to 1 ml Sircol dye reagent, followed by 30 minutes of mixing. After centrifugation at 10,000 g for 10 minutes, the supernatant was aspirated, and 1 ml of alkali reagent was added. Samples and collagen standards were then read at 540 nm in a spectrophotometer (Bio-Rad). Collagen concentrations were calculated using a standard curve with acid-soluble type I collagen.

Quantitative morphometry: To assess air space enlargement, the mean chord length (MCL) was quantified by superimposing a line grid on the images of lung sections at a magnification of 200x. Points on the lines of the grid hitting the air spaces, and intercepts of the lines with alveolar septa were counted to calculate MCL according to the formula: MCL = Σ Pair x L(p) / (Isepta / 2) (µm), where Σ Pair is the sum of the points of the grid overlaid air spaces, L(p) is the line length per point and Isepta is the sum of the intercepts of alveolar septa with the lines of the grid. For the morphometric assessment of alveolar surface area per unit volume of lung parenchyma (Sv) were determined by counting the number of points that fell on alveolar septal tissue and alveolar space, and by counting the number of intercepts with alveolar septal surface at a magnification of 200x according to the following formulas: Sv = 2 x Isepta / (Σ Ppar x L(p)/3) (1/µm), where Σ Ppar is the sum of the points of hitting parenchyma, L(p) is the line length per test point in µm, and Isepta the sum of the intercepts with alveolar septa.

**Table 1. Primer sequences and amplicon sizes for human tissues. All primer sets worked under identical quantitative PCR cycling conditions with similar efficiencies to obtain simultaneous amplification in the same run. Sequences were taken from GeneBank, all accession numbers are denoted.**

| Gene | Accession | Sequences (5'-*3') | | Length | Amplicon | SEQ ID NO: |
|---|---|---|---|---|---|---|
| *β-catenin* | NM001904 | for | *AAGTGGGTGGTATAGAGGCTCTTG* | 24 bp | 77 bp | 1 |
| | | rev | *GATGGCAGGCTCAGTGATGTC* | 21 bp | | 2 |
| *FZD1* | NM003505 | for | *AGCGCCGTGGAGTTCGT* | 17 bp | 64 bp | 3 |
| | | rev | *CGAAAGAGAGTTGTCTAGTGAGGAAAC* | 27 bp | | 4 |
| *FZD2* | NM001466 | for | *CACGCCGCGCATGTC* | 15 bp | 63 bp | 5 |
| | | rev | *ACGATGAGCGTCATGAGGTATTT* | 23 bp | | 6 |
| *FZD3* | NM017412 | for | *GGTGTTCCTTGGCCTGAAGA* | 20 bp | 72 bp | 7 |
| | | rev | *CACAAGTCGAGGATATGGCTCAT* | 23 bp | | 8 |
| *FZD4* | NM012193 | for | *GACAACTTTCACACCGCTCATC* | 22 bp | 164 bp | 9 |
| | | rev | *CCTTCAGGACGGGTTCACA* | 19 bp | | 10 |
| *GSK-3β* | NM002093 | for | *CTCATGCTCGGATTCAAGCA* | 20 bp | 86 bp | 11 |
| | | rev | *GGTCTGTCCACGGTCTCCAGTA* | 22 bp | | 12 |
| *LBF1* | NM016269 | for | *CATCAGGTACAGGTCCAAGAATGA* | 24 bp | 93 bp | 13 |
| | | rev | *GTCGCTGCCTTGGCTTTG* | 18 bp | | 14 |
| *LRP6* | NM002336 | for | *GATTCAGATCTCCGGCGAATT* | 21 bp | 83 bp | 15 |
| | | rev | *GGCTGCAAGATATTGGAGTCTTCT* | 24 bp | | 16 |
| *TCF3* | NM031283 | for | *ACCATCTCCAGCACACTTGTCTAATA* | 26 bp | 71 bp | 17 |
| | | rev | *GAGTCAGCGGATGCATGTGA* | 20 bp | | 18 |
| *TCF4* | NM030756 | for | *GCGCGGGATAACTATGGAAAG* | 21 bp | 89 bp | 19 |
| | | rev | *GGATTTAGGAAACATTCGCTGTGT* | 24 bp | | 20 |
| *WNT2* | NM003391 | for | *CCTGATGAATCTTCACAACAACAGA* | 25 bp | 78 bp | 21 |
| | | rev | *CCGTGGCACTTGCACTCTT* | 19 bp | | 22 |
| *WNT3a* | NM033131 | for | *GCCCCACTCGGATACTTCTTACT* | 23 bp | 98 bp | 23 |
| | | rev | *GAGGAATACTGTGGCCCAACA* | 21 bp | | 24 |
| *WNT7b* | NM058238 | for | *GCAAGTGGATTTTCTACGTGTTTCT* | 25 bp | 65 bp | 25 |
| | | rev | *TGACAGTGCTCCGAGCTTCA* | 20 bp | | 26 |
| *WNT7b* | NM003394 | for | *GCGCCAGGTGGTAACTGAA* | 19 bp | 59 bp | 27 |
| | | rev | *TGCCTGATGTGCCATGACA* | 19 bp | | 28 |
| *HPRT1* | NM00194 | for | *AAGGACCCCACGAAGTGTTG* | 20 bp | 137 bp | 29 |
| | | rev | *GGCTTTGTATTTTGCTTTTCCA* | 22 bp | | 30 |

**Table 2. Primer sequences and amplicon sizes for mouse tissues. All primer sets worked under identical quantitative PCR cycling conditions with similar efficiencies to obtain simultaneous amplification in the same run. Sequences were taken from GeneBank, all accession numbers are denoted.**

| **Gene** | **Accession** | **Sequences (5'—>>-3')** | | **Length** | **Amplicon** | **SEQ ID** NO |
|---|---|---|---|---|---|---|
| β*-catenin* | NM007614 | for | *TCAAGAGAGCAAGCTCATCATTCT* | 24 bp | 115 bp | 31 |
| | | rev | *CACCTTCAGCACTCTGCTTGTG* | 22 bp | | 32 |
| *AP5* | NM009701 | for | *CCTTATCCATTGGCTTGTCG* | 20bp | 115bp | 33 |
| | | rev | *CTGAACCGATTCATGACCAC* | 20bp | | 34 |
| *CDH1* | NM009864 | for | *CCATCCTCGGAATCCTTGG* | 19bp | 89bp | 35 |
| | | rev | *TTTGACCACCGTTCTCCTCC* | 20bp | | 36 |
| *Collal* | NM007742 | for | *CCAAGAAGACATCCCTGAAGTCA* | 23bp | 128bp | 37 |
| | | rev | *TGCACGTCATCGCACACA* | 18bp | | 38 |
| *FZD1* | NM021457 | for | *AAACAGCACAGGTTCTGCAAAA* | 22 bp | 58 bp | 39 |
| | | rev | *TGGGCCCTCTCGTTCTT* | 17 bp | | 40 |
| *FZD2* | NM020510 | for | *TCCATCTGGTGGGTGATTCTG* | 21 bp | 66 bp | 41 |
| | | rev | *CTCGTGGCCCCACTTCATT* | 19 bp | | 42 |
| *GSK-3β* | NM019827 | for | *TTTGAGCTGGATCCCTAGGATGA* | 23 bp | 75 bp | 43 |
| | | rev | *TTCTTCGCTTTCCGATGCA* | 19 bp | | 44 |
| *LEF1* | NM010703 | for | *GGCGGCGTTGGACAGAT* | 17 bp | 67 bp | 45 |
| | | rev | *CACCCGTGATGGGATAAACAG* | 21 bp | | 46 |
| *LRP5* | NM008513 | for | *CAACGTGGACGTGTTTTATTCTTC* | 24 bp | 138 bp | 47 |
| | | rev | *CAGCGACTGGTGGTGCTGTAGTCA* | 24 bp | | 48 |
| *LRP6* | NM008514 | for | *CCATTCCTCTCACTGGTGTCAA* | 22 bp | 146 bp | 49 |
| | | rev | *GCCAAACTCTACCACATGTTCCA* | 23 bp | | 50 |
| *PBGD* | NM013551 | for | *GGTACAAGGCTTTCACGATCGC* | 22 bp | 135bp | 51 |
| | | rev | *ATGTCCGGTAACGGCGGC* | 18 bp | | 52 |
| *SP-C* | NM001128660 | for | *TCTGCTCATGGGCCTCCAC* | 19 bp | 116bp | 53 |
| | | rev | *CGATGGTGTCTGCTCGCTC* | 19 bp | | 54 |
| *TCF4* | NM009333 | for | *GTGGGAACTGCCCCGTTT* | 18 bp | 59 bp | 55 |
| | | rev | *GTTCCTGATGAACCTTCACAAC* | 22 bp | | 56 |
| *TTF* | NM009442 | for | *AGCTTCCGAAGCCGAAGTATC* | 21 bp | 98bp | 57 |
| | | rev | *AGAACGGAGTCGTGTGCTTTG* | 21 bp | | 58 |
| *WNT2* | NM023653 | for | *AGCCCTGATGAACCTTCACAAC* | 22 bp | 78 bp | 59 |
| | | rev | *TGACACTTGCATTCTTGTTTCAAG* | 24 bp | | 60 |
| *WNT3a* | NM009522 | for | *GCACCACCGTCAGCAACA* | 18 bp | 57 bp | 61 |
| | | rev | *GGGTGGCTTTGTCCAGAACA* | 20 bp | | 62 |
| *WNT7b* | NM009528 | for | *TCGAAAGTGGATCTTTTACGTGTTT* | 25 bp | 67 bp | 63 |
| | | rev | *TGACAATGCTCCGAGCTTCA* | 20 bp | | 64 |
| *WNT10b* | NM011718 | for | *TGGGACGCCAGGTGGTAA* | 18 bp | 60 bp | 65 |
| | | rev | *CTGA CGTTCCA TGGCA TTTG* | 20 bp | | 66 |

### II. RESULTS

### 1. WNT/β-catenin signaling in human chronic obstructive pulmonary disease (COPD)

Initially, we sought to investigate whether components of the WNT/β-catenin signaling pathway are differentially regulated in human COPD. The mRNA level of the main WNT/β-catenin signaling components were quantified in lung tissue specimen of transplant donors and COPD patients (GOLD IV) using quantitative (q)RT-PCR. Samples underwent pathological examination and were taken from emphysematous areas. As depicted in Figure 1A, WNT ligands were variably expressed in the human adult lung, with low expression for *WNT10b.* In COPD lung specimens, only *WNT10b* mRNA levels were statistically significant upregulated (log-fold change of 2.23 ± 0.64). Next, we analyzed the expression of common WNT receptors and co-receptors. As shown in Figure 1B, the most abundant receptors in the human lung were frizzled (*FZD*) *1* and *4*, and the co-receptor low density lipoprotein receptor-related protein (*LRP*) 6. Interestingly, all receptors exhibited similar expression pattern in COPD and transplant donor lungs. Likewise, the intracellular mediators glycogen synthase kinase (*GSK*)-3β and *β-catenin* were equally expressed in COPD and transplant donor lungs (Figure 1C). All members of the T-cell-specific transcription factor/lymphoid enhancer-binding factor TCF/LEF family of transcription factors, except *TCF1,* were expressed but not differentially regulated in COPD or transplant donor lungs, respectively (Figure 1C).

We went on to specifically determine WNT/β-catenin signaling activity in COPD by phosphorylation analysis of LRP6 and GSK-3β, which has recently been demonstrated to be a sensitive indicator of WNT activity in tissue sections (25, 26). As shown in Figure 2, Western Blot analysis of phosphorylated GSK-3β and total GSK-3β, phosphorylated LRP6 and total LRP6, as well as active β-catenin and total β-catenin revealed that there was no difference between COPD and transplant donor samples. Densitometric analysis further confirmed these results, demonstrated no significant differences in optical density (OD) for phospho-GSK-3β (1.22±0.06 *vs.* 1.31±0.03; p=0.350) or active β-catenin (1.06±0.12 vs. 1.27±0.07; p=0.290) (Figure 2B). Densitometric analysis of phospho-LRP6 could not be assessed due to qualitative limitations. Expression of surfactant protein-C (SP-C) was analyzed as a positive control and revealed significant lower expression in COPD lung specimen (0.58±0.08 vs. 0.26±0.05; p=0.029).

Furthermore, we performed immunohistochemical analysis of WNT3a and β-catenin to reveal cells capable of WNT/β-catenin signaling in COPD lung tissue. As demonstrated by co-localization with SP-C, both, WNT3a (Figure 3A, B) and β-catenin (Figure 3C, D) expression was mainly detected in alveolar epithelial type II (ATII) cells in COPD and transplant donor lung tissue. Nuclear β-catenin staining, further indicating active WNT/β-catenin signaling was not detected in COPD lung tissue.

In summary, WNT/β-catenin signaling components were expressed in the alveolar epithelium in human lung tissue derived from COPD patients, but neither differentially regulation nor active signaling was detected in COPD. We conclude that the WNT/β-catenin signaling is silenced in COPD.

### 2. WNT/β-catenin signaling in experimental pulmonary emphysema

The results obtained thus far were obtained from human lung tissue derived from end-stage COPD patients. To gain am more comprehensive view about the role of WNT/β-catenin signaling and modulation thereof in earlier stages of this disease, we proceeded our studies using the mouse model of elastase-induced emphysema. Mice were subjected to a single orotracheal application of elastase and developed emphysema over a two-week time course (Figure 4A). As depicted in Figure 4, we analyzed in detail the mRNA expression profile of the main WNT/β-catenin signaling components during emphysema development in elastase-treated mice compared with their respective controls. All investigated WNT/β-catenin signaling components were expressed in emphysematous lungs, and, most importantly, exhibited mainly lower expression in elastase-treated mice compared with their respective controls, however significance was not reached for at all time points investigated. Significant downregulation was observed for *WNT2* and *WNT10b* as early as 1 day after induction (log-fold change day 1 for *WNT2:* 1.33±0.10; p<0.0001 and *WNT10b:* -1.26±0.35; p=0.0064) (Figure 4B). Further, *LRP6* and *FZD1* exhibited significantly decreased expression after emphysema induction at day 1 and day 7, respectively (log-fold change day 1 *LRP6:* -1.04±0.22; p=0.0056, day 7 *FZD1:* -1.78±0.20; p=0.0043) (Figure 4C). In addition, the transcription factors *LEF1* was significantly downregulated as early as day 1 (log-fold change -1.85±0.37; p=0.0036), while *TCF4* was significantly downregulated on day 7 (log-fold change -1.58±0.32; p=0.0172, Figure 4D). Importantly, downregulation of most investigated WNT/β-catenin signaling components was observed up to 7 days after emphysema induction, with expression levels returning to baseline after 14 days (Figure 4B - D), suggesting that active silencing of WNT signaling may take part in early emphysema development. Moreover, WNT reporter mice (TOPGAL) subjected to elastase treatment further corroborated these results, as no active WNT signaling has been observed (*data not shown).*

### 3. Activation of WNT/β-catenin signaling as a preventive and therapeutic approach in experimental emphysema

Next, we assessed, whether WNT/β-catenin activation represent an effective therapeutic option in emphysema. We used lithium chloride (LiCl), which mimics members of the WNT family by inhibiting the activity of GSK-3β, which causes intracellular accumulation of β-catenin (23). Under these circumstances, β-catenin can enter the nucleus and influence the expression of target genes. We applied LiCl to mice subjected to orotracheal instillation of elastase. First, we examined, whether WNT/β-catenin activation was preventive for the development of experimental emphysema *in vivo,* and started LiCl treatment on the day of emphysema induction followed by daily application for 7 days (referred to as "preventive approach", cf. detailed treatment scheme). In order to elucidate the effects of WNT/β-catenin activation on emphysema development, we analyzed key features of emphysema, such as destroyed lung architecture and surface, decreased alveolar epithelial type II (ATII) cells, or enhanced collagen deposition. Notably, histological assessment of the lung structure revealed marked attenuated emphysematous changes after preventive WNT/β-catenin activation in experimental lung emphysema (Figure 5). Further, immunohistochemical investigation demonstrated increased appearance of SP-C-positive cells with enhanced staining of the WNT/β-catenin signaling component WNT3a as well as nuclear β-catenin staining compared with vehicle-treated mice (Figure 6A, right panel, arrows). Furthermore, LiC1 treatment led to an increased SP-C protein expression (0.36±0.06 vs. 0.62±0.05; p=0.0231), suggesting an improved alveolar structure due to WNT/β-catenin activation in experimental emphysema (Figure 6B and C). Quantitative morphometric analysis of airspace enlargement further confirmed the beneficial effects of WNT/β-catenin activation by depicting improvement of elastase-induced changes. While the mean chord length was decreased (109.9±7.8 µm vs. 74.0±6.6 µm; p=0.0021, Figure 7A), the surface area of mice was increased (560.7±55.7 cm² vs. 317.8±27.5 cm²; p=0.0067, Figure 7B) in LiC1-treated mice compared with vehicle-treated mice. Moreover, total collagen content in lung homogenates was decreased in emphysematous mice treated with LiC1 compared with vehicle-treated mice (29.6µg/ml ± 4.1 vs. 16.1µg/ml + 3.9, p=0.042, Figure 7C), which was further substantiated by significantly reduced mRNA of type I collagen α1 in LiCl-treated animals (log-fold change -0.86±0.33; p=0.0496, Figure 7D). To further delineate the functional impact of WNT/β-catenin activation on alveolar structure, we assessed the mRNA levels of several alveolar epithelial cell marker (Figure 7D). As shown in Figure 5B and 7, the expression *SP-C* was increased in LiC1-treated mice (log-fold change - 1.21±0.42; p=0.0475). Furthermore, elevated mRNA level of cadherin 1 (*cdh1*), thyroid transcription factor 1 (*ttf1*)*,* forkhead box P2 (*foxp2*), and aquaporin 5 (*aqp5*) were observed after WNT/β-catenin activation in experimental emphysema (log-fold change *cdh1* 0.97±0.20, p=0.003; *ttf1* 1.20±0.30, *p=0.033; foxp2* 1.38±6.59, p=0.146; and *aqp5* 0.63±0.18, p=0.025, Figure 7D). In summary, preventive WNT/β-catenin activation led to an attenuation of experimental emphysema, as measured by decreased airspace enlargement, gas exchange area, and improved alveolar structure upon WNT activation.

Finally, we wanted to evaluate, whether WNT/β-catenin activation is able to induce lung regeneration after emphysema establishment. Therefore, we initiated LiC1 treatment on the day 7 after emphysema induction followed by daily application up to day 14 (referred to as "therapeutic approach", cf. detailed treatment scheme). Histological assessment of the lung structure revealed a marked attenuation of emphysema along with restored lung architecture (Figure 8A) and significantly decreased airspace enlargement after therapeutic WNT/β-catenin activation (mean chord length: 106.4±2.3 vs. 85.3±2.5; p=0.021, Figure 8B).

### III. DISCUSSION

COPD is a progressive and devastating disease and patients diagnosed with COPD have only limited therapeutic options (3, 27). COPD is characterized by irreversible expiratory airflow limitation due to two main intrapulmonary features: small airways disease (SAD) and emphysema. SAD includes airway inflammation with increased mucus production, airway wall remodeling, and peribronchiolar fibrosis, while emphysema is defined as destruction of the alveolar architecture due to distal airspace enlargement (28, 29). Cigarette smoking is the most important risk factor for COPD, and believed to activate several signal cascades, which impair the cellular and molecular maintenance, finally leading to destruction of the alveolar structure (30, 31). Strong evidence support a major role of inflammatory processes and protease / antiprotease imbalance in the development of emphysema, however, recent studies point out that additional or complimentary pathway abnormalities most probably are involved in the development and progression of alveolar destruction (14, 32). Along this line, developmentally active pathways have been suggested (33). Active signaling of the WNT/β-catenin signaling pathway during lung development has been reported (34, 35). Importantly, a recent study demonstrated that mouse embryos deficient for WNT2/2b expression display complete lung agenesis, further highlighting the importance of this signal pathway in lung morphogenesis (22).

Here, we took a comprehensive approach including the quantitative assessment of canonical WNT signaling components at the mRNA and protein level, localization, as well as activity of WNT ligands, receptors, and intracellular signaling molecules in human COPD specimen. Here, we demonstrated for the first time that WNT/β-catenin signaling is inactive and silenced in COPD.

Recently, the WNT/β-catenin pathway has been linked with parenchymal lung diseases, such as pulmonary fibrosis (36). Canonical WNT/β-catenin activation has been demonstrated to be activated in idiopathic pulmonary fibrosis (IPF), and, in particular, to be involved in epithelial cell repair mechanism *in vitro* and *in vivo* (18, 21). The finding that WNT/β-catenin signaling is silenced in COPD, further substantiate a critical role of WNT/β-catenin signaling in alveolar epithelial cell homeostasis, and reveal a reasonable mechanism for alveolar tissue destruction in emphysema.

It has to be pointed out, however, that the investigated human COPD tissue was obtained from patients undergoing lung transplantation, thereby presenting end-stage diseased lungs (GOLD IV). At this stage, efforts of the lung to repair or reverse may be diminished or even extinct. Early intervention therapy is unquestioned and patients suffering from COPD would benefit from immediate therapy. Thus, to further evaluate the impact of WNT/β-catenin signaling during emphysema development, and to be able to therapeutically intervene in a reasonable time frame, we continued our studies using the elastase-induced emphysema model in mice (37).

In our experimental emphysema model, the WNT/β-catenin signaling pathway was downregulated and not activated, as assessed by detailed expression and activity analysis of WNT/β-catenin components. Manifest changes in lung architecture, in particular during the first seven days, were accompanied by significant decreased expression of the most important components of WNT/β-catenin signaling. Following these observations, we aimed to assess whether WNT/β-catenin activation represent an effective therapeutic option in emphysema. To this end, we used lithium chloride (LiC1), which is well-known and described to increase WNT/β-catenin signaling by inhibition of glycogen synthase kinase 3β (GSK-3β) (23). Notably, we decided to pursue two different approaches, preventive as well as therapeutic WNT/β-catenin activation. Most importantly, WNT/β-catenin activation resulted in a marked attenuation of airspace enlargement and improvement of lung morphology in both treatment regimens.

WNT/β-catenin activation led to a reduction of collagen on the protein as well as mRNA level. Higher collagen content in the elastase-induced emphysema model was initially found in hamsters and has been shown to correlate with the degree of parenchyma destruction (38, 39). Increased collagen level after elastase administration have been suggested to present inefficient tissue repair attempts, implicating that decreased collagen level after WNT/β-catenin activation may be reflecting an improved tissue maintenance. Moreover, WNT/β-catenin activation led to a marked increase in several alveolar epithelial type (AT) II and I cell marker, such as SP-C and TTF1 (ATII), as well as AQP-5 (ATI), suggesting an increased maintenance of the alveolar structure due to WNT/β-catenin activation. Surfactant proteins play an important role in the maintenance of alveolar structure and impairment in surfactant metabolism in alveolar type II cells may lead to enlargement and destruction of the alveolar space (14, 40). Increased expression of AQP5, which is a water channel that resides in the ATI cell apical plasma membrane (41), further underlines that alveolar structure is restored after WNT/β-catenin activation in experimental emphysemsa. Recent studies highlighted a possible role of ATII and ATI cell apoptosis as a contributing mechanism to emphysema development (42, 43). The inability of activating WNT/β-catenin signaling in the alveolar epithelium in emphysema represent a crucial mechanism initiating or potentiating the loss of alveolar epithelial cells in emphysema.

Of note, future studies would benefit from the usage of different animal models, such as cigarette smoke exposure. Although the elastase- and smoke-induced models share similar mechanisms, such as inflammation and matrix remodeling, the smoke-induced model reflects the human disease more closely (37), and would most probably extend our insight on WNT/β-catenin signaling in COPD and emphysema.

In this study, we focused our investigations on one main characteristic feature of COPD: emphysema. Next to emphysema, COPD patients suffer from small airway disease, which is characterized by airway inflammation and peribronchiolar fibrosis. Active WNT/β-catenin signaling has been reported in other fibrotic disorders and in airway smooth muscle cell proliferation (44). Interestingly, it has been suggested that other mediators, such as transforming growth factor-β, may differ in the spatio-temporal expression, thereby contributing to the heterogeneity observed in COPD (11). Future studies focusing on small airway disease are needed to elucidate the involvement of WNT/β-catenin signaling in these processes. Similarly, the role of WNT signaling in lung cancer needs to be assessed carefully, in particular for future therapeutic developments. COPD patients exhibit a 4.5 fold increased risk for the development of lung cancer (45). Several WNT proteins have been found to be differentially expressed in non-small cell lung carcinoma specimen, apparently taken part in the multi-step oncogenic process (17, 46).

Our data demonstrate evidence for an involvement of WNT/β-catenin signaling in alveolar epithelial maintenance and destruction. With respect to the multiple mechanisms involved in COPD development, the role of WNT/β-catenin signaling in lung inflammation needs to be discussed. In a recent paper, Lewis *et al.* analyzed the gene expression profile of twelve different mouse models of infection, allergy, or lung injury (47). Importantly, the inventors reported differential expression of the WNT/β-catenin signaling pathway only in the mouse model of bleomycin-induced lung fibrosis, but not in any other inflammatory lung disease models. Furthermore, it has been shown that active WNT/β-catenin signaling occur in the fibroproliferative phase after acute lung injury in a mouse model of oxidant-induced injury (48), supporting an involvement of WNT/β-catenin signaling in the resolution and regeneration phase after lung injury.

Taken together, this study demonstrates silenced WNT/β-catenin signaling in human COPD specimen and in experimental emphysema. Preventive as well as therapeutic WNT/β-catenin activation led to a significant reduction of experimental emphysema with restored alveolar epithelial structure and function.

### REFERENCES

1. Jemal A, Ward E, Hao Y, Thun M. Trends in the leading causes of death in the united states, 1970-2002. Jama 2005;294:1255-1259.
2. Sullivan SD, Ramsey SD, Lee TA. The economic burden of copd. Chest 2000; 117:5S-9S.
3. Barnes PJ. Future treatments for chronic obstructive pulmonary disease and its comorbidities. ProcAm Thorac Soc 2008;5:857-864.
4. Chen ZH, Kim HP, Ryter SW, Choi AM. Identifying targets for copd treatment through gene expression analyses. IntJChron Obstruct Pulmon Dis 2008;3:359-370.
5. Lamprecht B, Schirnhofer L, Kaiser B, Buist S, Studnicka M. Non-reversible airway obstruction in never smokers: Results from the austrian bold study. Respir Med 2008;102:1833-1838*.*
6. Walter R, Gottlieb DJ, O'Connor GT. Environmental and genetic risk factors and gene-environment interactions in the pathogenesis of chronic obstructive lung disease. Environ Health Perspect 2000; 108 Suppl 4:733-742,
7. Tuder RM, Yoshida T, Fijalkowka I, Biswal S, Petrache I. Role of lung maintenance program in the heterogeneity of lung destruction in emphysema. Proc Am Thorac Soc 2006;3:673-679.
8. Morris DG, Huang X, Kaminski N, Wang Y, Shapiro SD, Dolganov G, Glick A, Sheppard D. Loss of integrin alpha(v)beta6-mediated tgf-beta activation causes mmp12-dependent emphysema. Nature 2003;422:169-173.
9. de Boer WI, van Schadewijk A, Sont JK, Sharma HS, Stolk J, Hiemstra PS, van Krieken JH. Transforming growth factor betal and recruitment of macrophages and mast cells in airways in chronic obstructive pulmonary disease. Am J Respir Crit Care Med 1998;158:1951-1957.
10. Bonniaud P, Kolb M, Gait T, Robertson J, Robbins C, Stampfli M, Lavery C, Margetts PJ, Roberts AB, Gauldie J. Smad3 null mice develop airspace enlargement and are resistant to tgf-beta-mediated pulmonary fibrosis. J Immunol 2004;173:2099-2108.
11. Morty RE, Konigshoff M, Eickelberg O. TGF-fi signaling across the ages: From distorted lung development to chronic obstructive pulmonary disease. Proc Am Thome Soc. 2009, in press.
12. Minai OA, Benditt J, Martinez FJ. Natural history of emphysema. Proc Am Thorac Soc 2008;5:468-474.
13. Thorley AJ, Tetley TD. Pulmonary epithelium, cigarette smoke, and chronic obstructive pulmonary disease. IntJ Chron Obstruct Pulmon Dis 2007;2:409-428.
14. Sharafkhaneh A, Hanania NA, Kim V. Pathogenesis of emphysema: From the bench to the bedside. Proc Am Thorac Soc 2008;5:475-477.
15. Taraseviciene-Stewart L, Voelkel NF. Molecular pathogenesis of emphysema. J Clin Invest 2008;118:394-402.
16. Moon RT, Kohn AD, De Ferrari GV, Kaykas A. Wnt and beta-catenin signalling: Diseases and therapies. Nat Rev Ge«ef 2004;5:691-701.
17. Konigshoff M, Eickelberg O. Wnt signaling in lung disease: A failure or a regeneration signal? Am JRespir Cell Mol Biol 2009., in press.
18. Konigshoff M, Kramer M, Balsara N, Wilhelm J, Amarie OV, Jahn A, Rose F, Fink L, Seeger W, Schaefer L, et al. Wntl-inducible signaling protein-1 mediates pulmonary fibrosis in mice and is upregulated in humans with idiopathic pulmonary fibrosis. J Clin Invest 2009; 119:772-787.
19. Logan CY, Nusse R. The wnt signaling pathway in development and disease. Annu Rev Cell Dev Biol 2004;20:781-810.
20. Cadigan KM, Liu YI. Wnt signaling: Complexity at the surface. J Cell Sci 2006;119:395-402.
21. Konigshoff M, Balsara N, Pfaff EM, Kramer M, Chrobak I, Seeger W, Eickelberg 0. Functional wnt signaling is increased in idiopathic pulmonary fibrosis. PLoS One 2008;3:e2142.
22. Goss AM, Tian Y, Tsukiyama T, Cohen ED, Zhou D, Lu MM, Yamaguchi TP, Morrisey EE. Wnt2/2b and beta-catenin signaling are necessary and sufficient to specify lung progenitors in the foregut. Dev Cell 2009;17:290-298.
23. Hedgepeth CM, Conrad LJ, Zhang J, Huang HC, Lee VM, Klein PS. Activation of the wnt signaling pathway: A molecular mechanism for lithium action. Dev Biol 1997;185:82-91.
24. Fehrenbach H, Voswinckel R, Michl V, Mehling T, Fehrenbach A, Seeger W. Nyengaard JR. Neoalveolarisation contributes to compensatory lung growth following pneumonectomy in mice. Eur Respir J2008;31:515-522.
25. Bilic J, Huang YL, Davidson G, Zimmermann T, Cruciat CM, Bienz M, Niehrs C. Wnt induces lrp6 signalosomes and promotes dishevelled-dependent lrp6 phosphorylation. Science 2007;316:1619-1622.
26. Forde JE, Dale TC. Glycogen synthase kinase 3: A key regulator of cellular fate. Cell Mol Life Sci 2007;64:1930-1944.
27. Viegi G, Pistelli F, Sherrill DL, Maio S, Baldacci S, Carrozzi L. Definition, epidemiology and natural history of copd. Eur Respir /2007;30:993-1013.
28. Hogg JC. Pathophysiology of airflow limitation in chronic obstructive pulmonary disease. Lancet 2004;364:709-721.
29. Macnee W. Pathogenesis of chronic obstructive pulmonary disease. Clin Chest Med 2007;28:479-513, v.
30. Tuder RM, Yoshida T, Arap W, Pasqualini R, Petrache I. State of the art. Cellular and molecular mechanisms of alveolar destruction in emphysema: An evolutionary perspective, Proc Am Thorac Soc 2006;3:503-510.
31. Rennard SI, Togo S, Holz O. Cigarette smoke inhibits alveolar repair: A mechanism for the development of emphysema. Proc Am Thorac Soc 2006;3:703-708.
32. Henson PM, Vandivier RW, Douglas IS. Cell death, remodeling, and repair in chronic obstructive pulmonary disease? Proc Am Thorac Soc 2006;3:713-717.
33. Shi W, Bellusci S, Warburton D. Lung development and adult lung diseases. Chest 2007;132:651-656.
34. Shu W, Jiang YQ, Lu MM, Morrisey EE. Wnt7b regulates mesenchymal proliferation and vascular development in the lung. Development 2002;129:4831-4842.
35. Cardoso WV, Lu J. Regulation of early lung morphogenesis: Questions, facts and controversies. Development 2006; 133:1611-1624.
36. Chilosi M, Poletti V, Zamo A, Lestani M, Montagna L, Piccoli P, Pedron S, Bertaso M: Scarpa A, Murer B, et al. Aberrant wnt/beta-eaten in pathway activation in idiopathic pulmonary fibrosis. Am JPathol 2003;162:1495-1502.
37. Wright JL, Cosio M, Churg A. Animal models of chronic obstructive pulmonary disease. Am J Physiol Lung Cell Mol Physiol 2008;295 :L 1 -15.
38. Lucey EC, Goldstein RH, Stone PJ, Snider GL. Remodeling of alveolar walls after elastase treatment of hamsters. Results of elastin and collagen mrna in situ hybridization. Am J Respir Crit Care Med 1998;158:555-564.
39. Rubio ML, Martin-Mosquero MC, Ortega M, Peces-Barba G, Gonzalez-Mangado N. Oral n-acetylcysteine attenuates elastase-induced pulmonary emphysema in rats. Chest 2004;125:1500-1506.
40. Yokohon N, Aoshiba K, Nagai A. Increased levels of cell death and proliferation in alveolar wall cells in patients with pulmonary emphysema. Chest 2004; 125:626-632.
41. Verkman AS. Role of aquaporins in lung liquid physiology. Respir Physiol Neurobiol 2007;159:324-330.
42. Yan C, Du H. Alveolus formation: What have we learned from genetic studies? J Appl Physiol 2004;97:1543-1548.
43. Yoshida T, Tuder RM. Pathobiology of cigarette smoke-induced chronic obstructive pulmonary disease. Physiol Rev 2007;87:1047-1082.
44. Nunes RO, Schmidt M, Dueck G, Baarsma H, Halayko AJ, Kerstjens HA, Meurs H. Gosens R. Gsk-3/beta-catenin signaling axis in airway smooth muscle: Role in mitogenic signaling. Am J Physiol Lung Cell Mol Physiol 2008;294:L1110-1118.
45. Houghton AM, Mouded M, Shapiro SD. Common origins of lung cancer and copd. Nat Med 2008; 14:1023-1024.
46. Mazieres J, He B, You L, Xu Z, Jablons DM. Wnt signaling in lung cancer. Cancer Lett 2005;222:1-10.
47. Lewis CC, Yang JY, Huang X, Banerjee SK, Blackburn MR, Baluk P, McDonald DM, Blackwell TS, Nagabhushanam V, Peters W, et al. Disease-specific gene expression profiling in multiple models of lung disease. AmJRespir Crit Care Med 2008;177:376-387.
48. Douglas IS, Diaz del Valle F, Winn RA, Voelkel NF. Beta-catenin in the fibroproliferative response to acute lung injury. Am JRespir Cell Mol Biol 2006;34:274-285.
49. Tian D, Zhu M, Li J, Ma Y, Wu R. Cigarette smoke extract induces activation of beta-catenin/TCF signaling through inhibiting GSK3beta in human alveolar epithelial cell line. Toxicol Lett. 2009;187:58-62.
50. EP 1 222 933 A1
51. Roth M, Connett J, D'Armiento J, Foronjy R, Friedman P, Goldin J, Louis T, Mao J, Muindi J, O'Connor G, Ramsdell J, Ries A, Scharf S, Schluger N, Sciurba F, Skeans M, Walter R, Wendt C, Wise R. Feasibility of retinoids for the treatment of emphysema study. Chest 2006;130 (5):1334-45.
52. Zhong H, Zou H, Semenov MV, Moshinsky D, He X, Huang H, Li S, Quan J, Yang Z, Lin S. Characterization and development of novel small-molecules inhibiting GSK3 and activating Wnt signaling. Mol Biosyst. 2009; 5(11):1356-60.
53. Herzog et al. 2008 Herzog EL, Brody AR, Colby TV, Mason R, Williams MC. Knowns and unknowns of the alveolus. Proc Am Thorac Soc. 2008; 5(7):778-82
54. Forde JE, Dale TC. Glycogen synthase kinase 3: A key regulator of cellular fate. Cell Mol Life Sci 2007;64(15):1930-1944.
55. Kim KH, Song MJ, Yoo EJ, Choe SS, Park SD, Kim JB. Regulatory role of glycogen synthase kinase 3 for transcriptional activity of ADD1/SREBP1c J Biol Chem. 2004;279(50):51999-2006.

## Claims

1. An inhibitor of glycogen synthase kinase 3 (GSK-3) for use in a method of treating or preventing emphysema.

2. The inhibitor for the use of claim 1, wherein the inhibitor is an inhibitor of glycogen synthase kinase 3β (GSK-3β).

3. The inhibitor for the use of claim 1 or 2, wherein the inhibitor is a low-molecular-weight chemical compound having a molecular weight of at most 2000 Da, preferably at most 1500 Da, more preferably at most 1000 Da, especially at most 800 Da.

4. The inhibitor for the use of any of claims 1 to 3, wherein the inhibitor is selected from the group consisting of Li⁺, GSK-3 inhibitor IX, GSK-3 inhibitor XII, GSK-3 inhibitor XV, SB-216763, SB-415286 and 3F8.

5. The inhibitor for the use of any of claims 1 to 4, wherein the inhibitor is for use in a method of preventing emphysema in a subject.

6. The inhibitor for the use of claim 5, wherein the subject is at risk of developing an emphysema due to smoke, alpha 1-antitrypsin deficiency, exposure to occupational chemicals and/or dust, indoor and outdoor air pollution, abnormal lung development, familial disposition, connective tissue disorders, or immune-related diseases, such as HIV.

7. A method of activating an emphysema cell, the method comprising
- contacting the cell with a GSK-3 inhibitor, thereby activating the cell.

8. The method of claim 7, wherein the GSK-3 inhibitor is further defined as in any of claims 1 to 4.

9. The method of claim 7 or 8, wherein the emphysema cell is a lung cell, particularly derived from the lung parenchyma, especially from the alveolar epithelium, such as an alveolar type I (ATI) and type II (ATII) cell, or interstitium (such as a fibroblast cell).

10. An activated emphysema cell obtained by a method of any of claims 7 to 9 for use in a method of treating or preventing emphysema in a subject.

11. The activated emphysema cell for the use of claim 10, wherein the cell is an autologous cell.

12. The activated emphysema cell for the use of claim 10 or 11, wherein the subject is at risk of developing an emphysema due to smoke, alpha 1-antitrypsin deficiency, exposure to occupational chemicals and/or dust, indoor and outdoor air pollution, abnormal lung development, familial disposition, connective tissue disorders, or immune-related diseases, such as HIV.

13. A method of screening for a substance for treating or preventing emphysema, the method comprising
- contacting a GSK-3 or functionally active derivative thereof with a compound; and
- determining the activity of the GSK-3 in the presence of the compound, wherein the compound is identified as a substance for treating or preventing emphysema, if the activity of GSK-3 in the presence of the compound is reduced relative to a control.

14. The method of claim 13, wherein the GSK-3 is GSK-3β.
